(19) Europäisches Patentamt
European Patent Office
Office européen des brevets

(11) EP 3 615 506 B1

(12) **FASCICULE DE BREVET EUROPEEN**

(45) Date de publication et mention de la délivrance du brevet:
**20.10.2021 Bulletin 2021/42**

(21) Numéro de dépôt: **18725283.8**

(22) Date de dépôt: **24.04.2018**

(51) Int Cl.:
*C07C 69/017* (2006.01) *C07C 69/612* (2006.01)
*C07C 69/708* (2006.01) *C07C 43/20* (2006.01)
*C09D 11/037* (2014.01) *C07C 69/34* (2006.01)
*C07C 69/44* (2006.01) *C07C 69/48* (2006.01)
*C07C 69/616* (2006.01) *C07C 69/618* (2006.01)
*C07C 69/736* (2006.01) *C09D 11/17* (2014.01)
*C09D 11/50* (2014.01)

(86) Numéro de dépôt international:
**PCT/FR2018/051031**

(87) Numéro de publication internationale:
**WO 2018/197807 (01.11.2018 Gazette 2018/44)**

(54) **NOUVEAUX COMPOSÉS DE FORMULE (I) ET LEUR UTILISATION DANS DES COMPOSITIONS DE PIGMENT THERMOCHROME**

NEUARTIGE VERBINDUNGEN VON FORMEL (I) UND VERWENDUNG DAVON IN THERMOCHROMEN PIGMENTZUSAMMENSETZUNGEN

NOVEL COMPOUNDS OF FORMULA (I) AND USE THEREOF IN THERMOCHROMIC PIGMENT COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.04.2017 FR 1753677**

(43) Date de publication de la demande:
**04.03.2020 Bulletin 2020/10**

(73) Titulaire: **Société BIC**
**92110 Clichy (FR)**

(72) Inventeurs:
- **DEBRAUWER, Christelle**
  **Quebec, G1S3T3 (CA)**
- **DAMIANO, Anne-Lise**
  **13470 Carnoux-en-Provence (FR)**
- **BOURQUE, Alexander**
  **77144 Montevrain (FR)**
- **FOULONNEAU, François**
  **33800 Bordeaux (FR)**
- **CHOLLET, Guillaume**
  **33850 Leognan (FR)**

(74) Mandataire: **Cabinet Beau de Loménie**
**158, rue de l'Université**
**75340 Paris Cedex 07 (FR)**

(56) Documents cités:
**EP-A1- 3 009 493** **WO-A1-2016/198784**
**US-A- 2 503 731**

- **AMORATI R ET AL: "Synthesis of new Cardanol and Cardol derivatives by allylation and regioselctive cyclocarbonylation reactions", SYNTHESIS, GEORG THIEME VERLAG, STUTTGART, DE, no. 18, 1 janvier 2002 (2002-01-01), pages 2749-2755, XP002576726, ISSN: 0039-7881, DOI: 10.1055/S-2002-35983**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouveaux composés comme définis dans les revendications et leur utilisation comme milieu réactionnel dans des compositions de pigment thermochrome. La présente invention vise également des microcapsules de pigment thermochrome comprenant de telles compositions de pigment thermochrome, des compositions d'encre comprenant de telles microcapsules de pigment thermochrome, et enfin des instruments d'écriture comprenant de telles compositions d'encre.

## ETAT DE L'ART ANTÉRIEUR ET BUT DE L'INVENTION

Les compositions de pigment thermochrome présentent des propriétés de décoloration réversible liées à un changement de température. Ces compositions trouvent application lorsqu'un marquage à l'encre exige des effaçages répétés.

L'effet thermochrome d'une encre fonctionne grâce à l'association des trois composés suivants :

(A) au moins un composé organique colorant donneur d'électrons ou leuco-colorant,
(B) au moins un composé accepteur d'électrons ou développeur de couleur, et
(C) au moins un composé servant de milieu réactionnel capable de conduire à une réaction d'acceptation/don d'électrons réversible attribuable aux composés (A) et (B) ou agent régulateur de changement de température.

Les changements de température provoquent réversiblement la coloration ou la décoloration des encres. Ainsi, l'augmentation de chaleur va provoquer l'effacement de l'encre, tandis qu'un refroidissement provoquera son apparition. Ces changements suivent le schéma de la **Figure 1.** Sur ce schéma, la température de début de disparition de la couleur de l'encre est T3, celle à laquelle la couleur de l'encre a totalement disparue est T4 et TG est la température médium entre T3 et T4. A l'inverse, la température de début de réapparition de la couleur de l'encre est T2, celle à laquelle la couleur de l'encre a totalement réapparu est T1 et TH est la température se trouvant au milieu entre T1 et T2. On appelle largeur d'hystérésis de changement de couleur ($\Delta H$), la plage entre (TH) et (TG).

De manière surprenante, les Inventeurs ont découverts de nouveaux composés permettant la préparation de microcapsules de pigment thermochrome présentant des plages de températures de fusion et de cristallisation optimales correspondant respectivement aux températures de décoloration et de recoloration de ces compositions. Les composés de l'invention présentent ainsi de nombreux avantages à être utilisés comme agent régulateur de changement de température dans des encres thermochromes : ils présentent des caractéristiques d'hystérésis remarquables et un contraste de couleurs extrêmement élevé entre l'état coloré et l'état décoloré. Les nouveaux composés de l'invention ont en outre l'avantage de pouvoir être préparé via un procédé écologique, c'est-à-dire à partir d'un produit recyclé et biosourcé, le 3-pentadécylphénol, provenant des déchets de la production des noix de cajou.

WO 2016/198784 A1 décrit des compositions de pigment thermochrome comprenant (A) au moins un composé colorant donneur d'électrons, (B) au moins un composé accepteur d'électrons, et (C) un mélange d'au moins un composé ester 4-benzyloxyphénylalkyle et au moins un triester de glycérol. Ces compositions présentent une faible largeur d'hystérésis.

## DESCRIPTION DE L'INVENTION

Selon un premier aspect, la présente invention a pour objet un composé répondant à la formule (I) suivante :

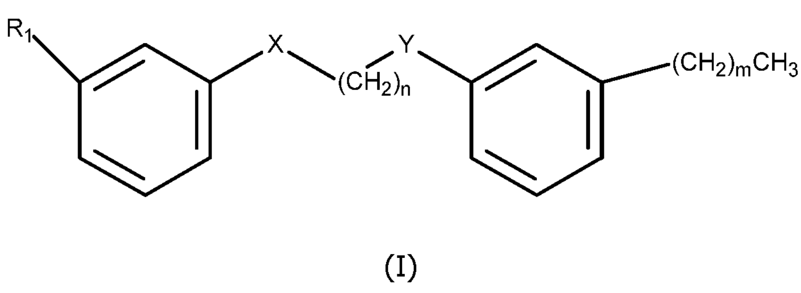

(I)

dans laquelle :

- X représente $CHR_2$, O ou OCO,
- Y représente O ou COO,

- $R_1$ représente H ou $(CH_2)_pCH_3$,
- $R_2$ représente un groupe phényle ou H,
- m = 12-18,
- n = 0-14,
- p = 12-18, et

à la condition que lorsque n = 0, X représente $CHR_2$.

Au sens de la présente invention :

- lorsque X = OCO: l'atome d'oxygène est attaché au groupe phényle et le groupe carbonyle à la chaîne $(CH_2)_n$, et
- lorsque Y = COO: le groupe carbonyle est attaché à la chaîne $(CH_2)_n$ et l'atome d'oxygène au groupe phényle.

Dans la formule (I) ci-dessus, X est de préférence choisi parmi $CHR_2$, O ou OCO.

Dans la formule (I) ci-dessus, m peut être indépendamment choisi parmi un des entiers suivants : 12, 13, 14, 15, 16, 17 ou 18.

Dans la formule (I) ci-dessus, n peut être indépendamment choisi parmi un des entiers suivants : 0, 1, 2, 3, 4, 5, 6, 7, 8, 9, 10, 11, 12, 13 ou 14.

Dans la formule (I) ci-dessus, p peut être indépendamment choisi parmi un des entiers suivants : 12, 13, 14, 15, 16, 17 ou 18.

Dans la formule (I) ci-dessus, de préférence n = 0-10.

Dans la formule (I) ci-dessus, de préférence p = 14.

Avantageusement, le composé de formule (I) de l'invention est un composé dans lequel :

- X représente $CHR_2$, O ou OCO,
- Y représente O ou COO,
- $R_1$ représente H ou $(CH_2)_pCH_3$,
- $R_2$ représente un groupe phényle ou H,
- m = 12-18,
- n = 0-14, de préférence n = 0-10, et
- p = 12-18, de préférence p = 14,

à la condition que lorsque n = 0, X représente $CHR_2$.

Selon un premier mode de réalisation préféré, le composé de l'invention répond à la formule ($I_a$) suivante :

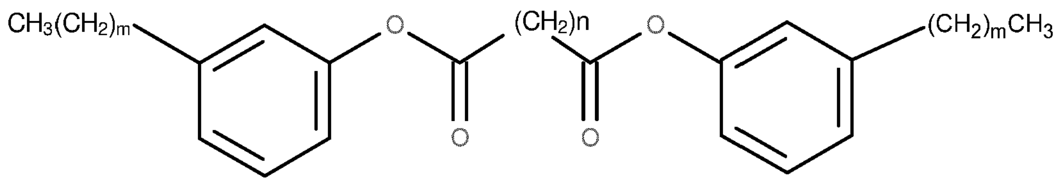

($I_a$)

dans laquelle :

- m = 12-18, et
- n = 1-14, de préférence n = 1-10.

Avantageusement, dans la formule ($I_a$) de l'invention : m = 14.

Avantageusement, dans la formule ($I_a$) de l'invention : n = 8.

Selon un deuxième mode de réalisation préféré, le composé de l'invention répond à la formule ($I_b$) suivante :

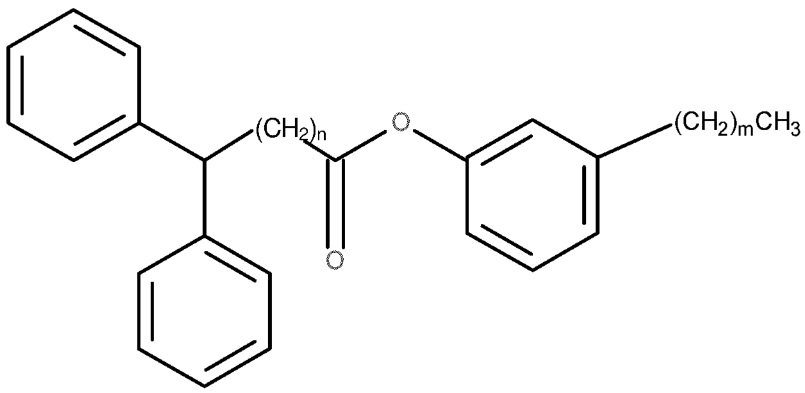

($I_b$)

dans laquelle :

- m = 12-18, et
- n = 0-14, de préférence n = 0-10.

Avantageusement, dans la formule ($I_b$) de l'invention : m = 14.
Avantageusement, dans la formule ($I_b$) de l'invention : n = 1.
Selon un troisième mode de réalisation préféré, le composé de l'invention répond à la formule ($I_c$) suivante :

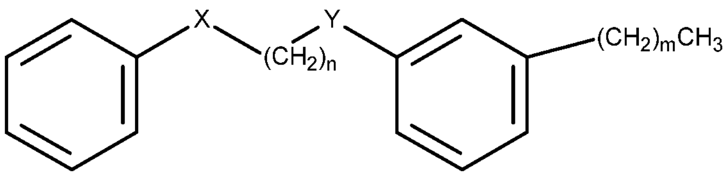

($I_c$)

dans laquelle :

- X représente $CH_2$ ou O,
- Y représente O ou COO,
- m = 12-18, et
- n = 1-14, de préférence n = 1-10, et encore plus préférentiellement n = 1-8.

Avantageusement, dans la formule ($I_c$) de l'invention : m = 14.
Avantageusement, dans la formule ($I_c$) de l'invention : n = 2.
Les composés de formule ($I_a$), ($I_b$) et ($I_c$) peuvent être synthétisés selon les deux premières voies de synthèse décrites ci-après. Le composé de formule ($I_c$) peut en outre être synthétisé selon la troisième voie de synthèse décrite ci-après.
La première voie de synthèse répond au schéma réactionnel suivant :

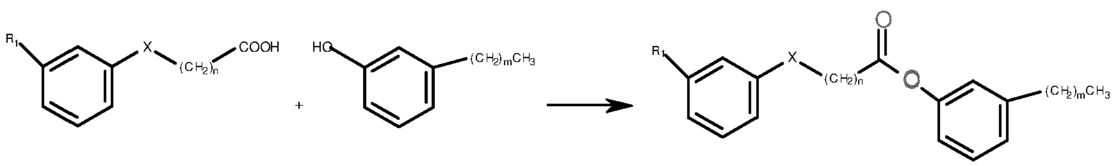

Dans cette première voie de synthèse, l'alcool joue le rôle de solvant. Il est utilisé en excès, de préférence à

un ratio alcool/acide carboxylique de 1,5/1 à 3/1, et encore plus préférentiellement de 2/1. Le mélange d'alcool et d'acide carboxylique est chauffé à une température allant de 120 à 200°C, de préférence de 140 à 160°C, sous pression réduite, de préférence entre 200 et 800 mbar, jusqu'à ce que l'acide soit totalement consommé. Le mélange est laissé chauffer, sous pression réduite, pendant une durée allant de 1 à 4 jours. Le composé de formule ($I_a$), ($I_b$) ou ($I_c$) obtenu est ensuite purifié par recristallisation.

La deuxième voie de synthèse répond au schéma réactionnel suivant :

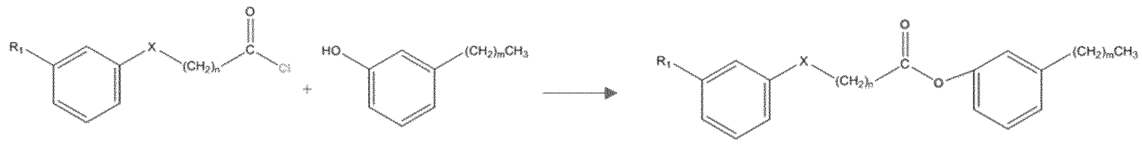

Dans cette deuxième voie de synthèse, le phénol est solubilisé avec un catalyseur dans un solvant polaire aprotique tel que le tétrahydrofuranne (THF). Le catalyseur est de préférence une base volatile comme la triéthylamine. Le mélange est maintenu à froid, une température allant de -20 à 30°C, préférentiellement de 0 à 20°C. Le mélange est maintenu à froid dans un bain de glace ou dans un bain de $CO_2$ solide plongé dans un solvant tel que l'acétone ou l'éthanol. Le mélange est avantageusement rendu inerte par ajout d'azote. Le chlorure d'acide est ensuite ajouté lentement, au goutte-à-goutte, pendant une durée allant de 15 à 60 minutes. Le ratio phénol/chlorure d'acide utilisé est de préférence de 1,1/1 à 1/1,1, et encore plus préférentiellement de 1/1. La température du mélange est ensuite élevée à température ambiante (25°C), et le mélange maintenu à cette température pendant 1 à 3 heures, de préférence 2 heures, sous agitation. Le composé de formule ($I_a$), ($I_b$) ou ($I_c$) obtenu est ensuite purifié par recristallisation.

Le composé de formule ($I_c$) peut également être synthétisé selon la voie de synthèse suivante :

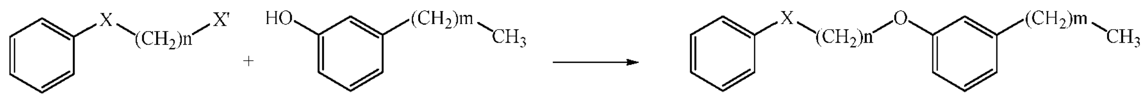

le substituant X' représentant un atome d'halogène choisi parmi Cl, Br, I ou F, et de préférence l'atome de Br.

Dans cette voie de synthèse, les composés de formule ($I_c$) peuvent être préparés :

- Soit par solubilisation des réactifs dans un solvant polaire tel que le *tert*-butanol, en présence d'une base forte telle que l'hydroxyde de sodium. Le ratio alcool/composé halogéné utilisé est de préférence de 1/1 à 1,4/1, et encore plus préférentiellement de 1,2/1. Le mélange de réactifs est chauffé à reflux, à une température allant de 75 à 90°C, pendant 2 à 10 heures, de préférence 6 heures. Le composé de formule ($I_c$) obtenu est ensuite purifié par recristallisation
- Soit par solubilisation des réactifs dans un solvant polaire aprotique tel que l'acétonitrile, en présence d'une base faible telle que le carbonate de potassium. Le ratio alcool/composé halogéné utilisé est de préférence de 1/1 à 1,4/1, et encore plus préférentiellement de 1,2/1. Le mélange de réactifs est chauffé à reflux, à une température allant de 75 à 90°C, pendant 5 à 10 jours, de préférence 7 jours. Le mélange est ensuite filtré pour éliminer la base faible. Le composé de formule ($I_c$) obtenu est ensuite purifié par recristallisation.

Le composé de formule (I) de l'invention est de préférence choisi parmi les composés suivants :

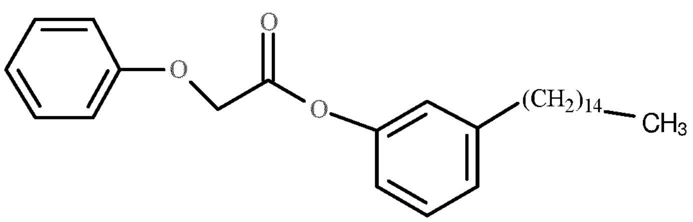

(1)

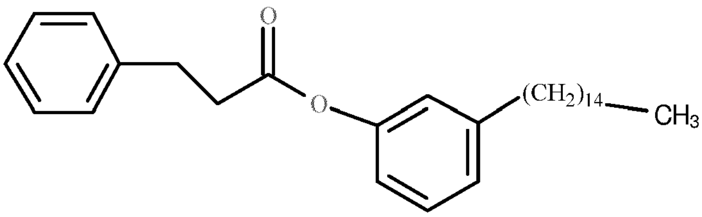

(3)

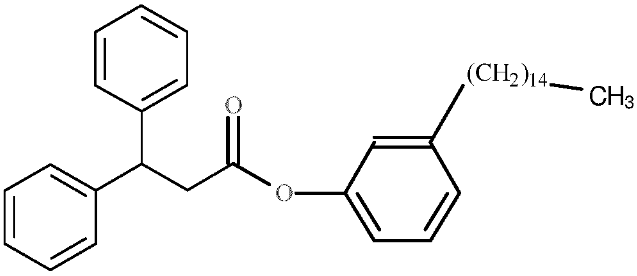

(4)

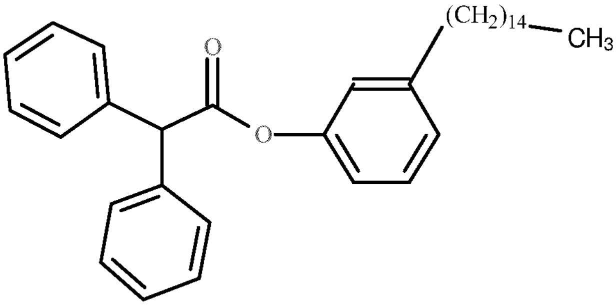

(5)

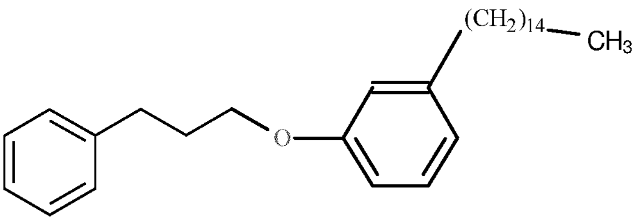

(6)

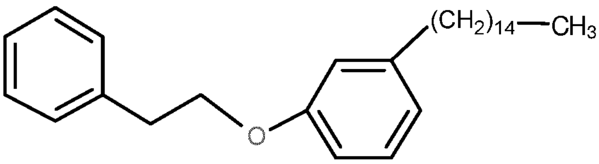

(7)

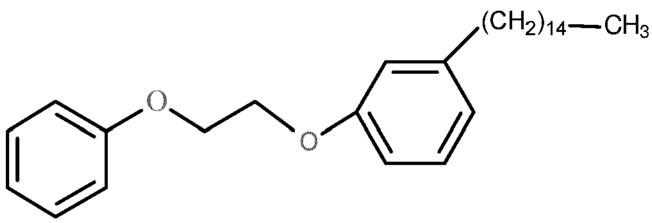

(8)

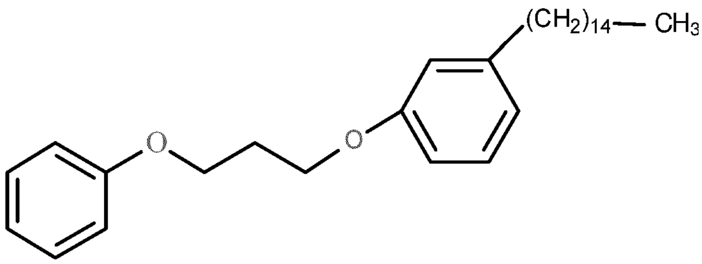

(9)

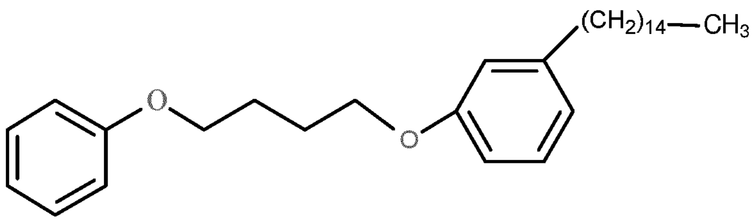

(10)

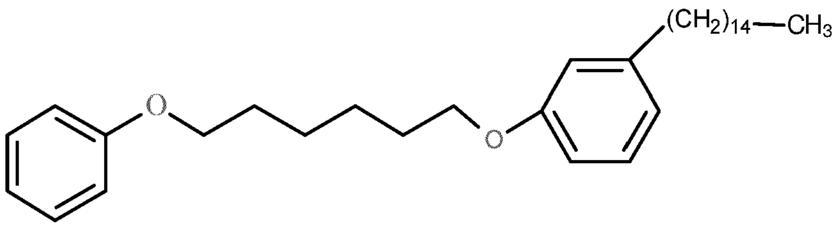

(11)

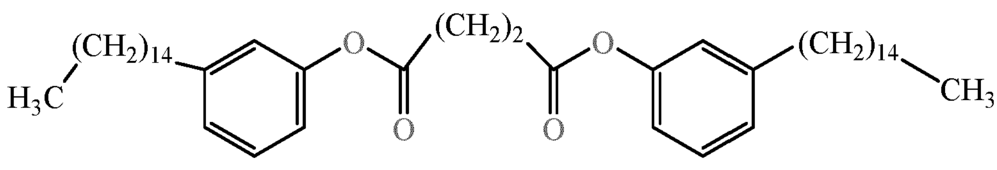

(12)

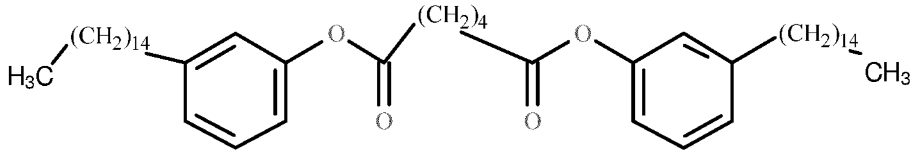

(13)

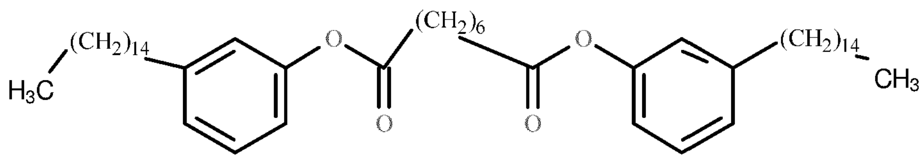

(14)

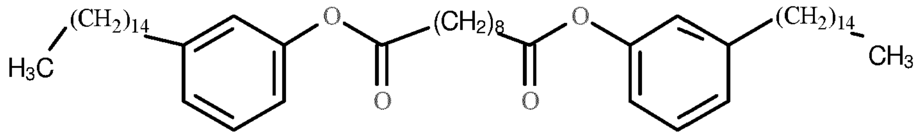

(15)

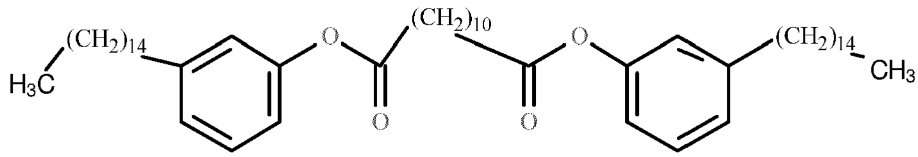

(16)

La température de fusion du composé de formule (I) de l'invention peut varier de 20 à 80°C, de préférence de 30 à 80°C, et encore plus préférentiellement de 40 à 70°C. C'est cette température de fusion optimale qui fait que le composé de formule (I) de l'invention est un composé idéal qui présente les propriétés requises pour être utilisé comme agent régulateur de changement de température dans des compositions de pigment thermochrome.

Ainsi, un autre objet de l'invention vise une composition de pigment thermochrome comprenant :

(A) au moins un composé organique colorant donneur d'électrons ou leuco-colorant,
(B) au moins un composé accepteur d'électrons ou développeur de couleur, et
(C) au moins un composé répondant à la formule (I) suivante :

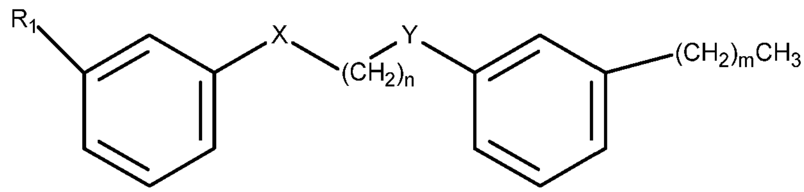

dans laquelle :

- X représente $CHR_2$, O, OCO ou CH=CH,
- Y représente O ou COO,
- $R_1$ représente H ou $(CH_2)_pCH_3$,
- $R_2$ représente un groupe phényle ou H,
- m = 12-18,
- n = 0-14,
- p = 12-18, et

à la condition que lorsque n = 0, X représente $CHR_2$ ou CH=CH.

Les taux en poids des composés (A), (B) et (C) sont influencés par la nature et la concentration de chacun de ces composés.

Le taux en poids de composé organique colorant donneur d'électrons (A) peut varier de 1 à 10%, de préférence de 1 à 6%, et encore plus préférentiellement de 2 à 4%, en poids par rapport au poids total de la composition de pigment thermochrome.

Le taux en poids de composé accepteur d'électrons (B) peut varier de 1 à 20%, de préférence de 1 à 14%, et

encore plus préférentiellement de 4 à 10%, en poids par rapport au poids total de la composition de pigment thermochrome.

Le taux en poids de composé (C) de formule (I) jouant le rôle de milieu réactionnel peut varier de 70 à 98%, de préférence de 80 à 98%, et encore plus préférentiellement de 86 à 94%, en poids par rapport au poids total de la composition de pigment thermochrome.

Ainsi, la composition de pigment thermochrome de l'invention peut comprendre :

(A) de 1 à 10%, de préférence de 1 à 6%, et encore plus préférentiellement de 2 à 4%, en poids d'au moins un composé organique colorant donneur d'électrons,
(B) de 1 à 20%, de préférence de 1 à 14%, et encore plus préférentiellement de 4 à 10%, en poids d'au moins un composé accepteur d'électrons, et
(C) de 70 à 98%, de préférence de 80 à 98%, et encore plus préférentiellement de 86 à 94%, en poids d'au moins un composé répondant à la formule (I).

Selon un mode de réalisation préféré, la composition de pigment thermochrome de l'invention comprend :

(A) de 2 à 4% en poids d'au moins un composé organique colorant donneur d'électrons,
(B) de 4 à 10% en poids d'au moins un composé accepteur d'électrons, et
(C) de 86 à 94% en poids d'au moins un composé répondant à la formule (I).

Avantageusement, la composition de pigment thermochrome de l'invention présente une largeur d'hystérésis de changement de couleur (ΔH) après encapsulation allant de 20 à 80°C, de préférence de 30 à 80°C, et encore plus préférentiellement de 40 à 70°C.

En tant que composé organique colorant donneur d'électrons (A), des composés classiquement connus tels que les phtalides de diphénylméthane, les phtalides phénylindolyl, les indolylphthalides, les azaphthalides de diphénylméthane, les azaphthalides de phénylindolyl, les fluoranes, les styrylquinolines et les lactones diazarhodamine peuvent être cités, des exemples de ces composés étant présentés ci-après.

Le composé organique colorant donneur d'électrons (A) peut ainsi être choisi parmi le 3-(4-diéthylamino-2-éthoxyphényl)-3-(l-éthyl-2-méthylindol-3-yl)-4-azaphthalide (Blue 63, n° CAS: 69898-40-4), le 3,3-bis(p-diméthylaminophényl)-6-diméthylaminophthalate (n° CAS: 1552-42-7), le 2'-chloro-6'-(diéthylamino)-3'-méthylfluorane (n° CAS: 21121-62-0), le 6'-(diéthylamino)-1',3'-diméthylfluorane (n° CAS : 21934-68-9), le 2-chloro-6-(diéthylamino)-fluorane (n° CAS : 26567-23-7), le 3-diéthylaminobenzofluorane (n° CAS: 26628-47-7), le 3',6'-bis(diéthylamino)-2-(4-nitrophényl)spiro[isoindole-l,9'-xanthène]-3-one (n° CAS: 29199-09-5), le 2-phénylamino-3-méthyl-6-diéthylaminofluorane (n° CAS: 29512-49-0), le 2'-(dibenzylamino)-6'-(diéthylamino)fluorane (n° CAS: 34372-72-0), le 2-(2,4-diméthylphénylamino)-3-méthyl-6-diéthylaminofluorane (Black 15, n° CAS: 36431-22-8), le 3-(1,2-diméthyl-3-indolyl)-3-[4-(diéthylamino)-2-méthylphényl]phthalide (n° CAS: 36499-49-7), le 3',6'-diméthoxyfluorane (n° CAS: 36886-76-7), le 3,3-bis-(1-butyl-2-méthyl-indol-3-yl)-3H-isobenzofuran-1-one (Red 40, n° CAS: 50292-91-6), le 3,3-bis-(2-méthyl-1-octyl-1H-indol-3-yl)-3H-isobenzofuran-1-one (n° CAS: 50292-95-0), le 2'-anilino-6'-[éthyl(p-tolyl)amino]-3'-méthylspiro[isobenzofuran-1(3H),9'-[9H]xanthène]-3-one (n° CAS: 59129-79-2), le 3-(N-éthyl-n-isopentylamino)-6-méthyl-7-anilino fluorène (n° CAS: 70516-41-5), le 3-[4-(diéthylamino)phényl]-3-(1-éthyl-2-méthyl-1H-indol-3-yl)phthalide (n° CAS: 75805-17-3), le 2'-(2-chloroanilino)-6'-(dibutylamino)fluorane (n° CAS: 82137-81-3), le 2-phénylamino-3-méthyl-6-dibutylaminofluorane (n° CAS: 89331-94-2), le 3-(1-butyl-2-méthyl-1H-indol-3-yl)-6-(diméthylamino)-3-[4-(diméthylamino)phényl]-3-(1(3H)-isobenzofuranone (n° CAS : 92453-31-1), le 7-(4-diéthylamino-2-hexyloxyphényl)-7-(1-éthyl-2-méthyl-1H-indol-3-yl)-7H-furo[3,4-b]pyridin-5-one (Blue 203, n° CAS: 98660-18-5), le 7,7-bis[4-(diéthylamino)-2-éthoxyphényl]furo[3,4-b]pyridin-5-one (n° CAS: 132467-74-4), le N,N-diméthyl-4-[2-[2-(octyloxy)phényl]-6-phényl-4-pyridinyl]benzènamine (Yellow CK37, n° CAS: 144190-25-0), le 3-(2,2-bis(1-éthyl-2-méthylindol-3-yl)vinyl)-3-(4-diéthylaminophényl)-phthalide (n° CAS : 148716-90-9).

De manière préférée, le composé organique colorant donneur d'électrons (A) est choisi parmi le 3-(4-diéthylamino-2-éthoxyphényl)-3-(1-éthyl-2-méthylindol-3-yl)-4-azaphthalide (Blue 63, n° CAS: 69898-40-4), le 2'-(dibenzylamino)-6'-(diéthylamino)fluorane (n° CAS: 34372-72-0), le N,N-diméthyl-4-[2-[2-(octyloxy)phényl]-6-phényl-4-pyridinyl]benzènamine (Yellow CK37, n° CAS: 144190-25-0), le 7-(4-diéthylamino-2-hexyloxyphényl)-7-(1-éthyl-2-méthyl-1H-indol-3-yl)-7H-furo[3,4-b]pyridin-5-one (Blue 203, N° CAS: 98660-18-5), le 2-(2,4-diméthylphénylamino)-3-méthyl-6-diéthylaminofluoran (Black 15, N° CAS : 36431-22-8), et le 3,3-bis-(1-butyl-2-méthyl-indol-3-yl)-3H-isobenzofuran-1-one (Red 40, n° CAS: 50292-91-6).

En tant que composé accepteur d'électrons (B), on peut citer à titre non limitatif les composés ayant un proton actif tels que les composés ayant un groupe hydroxyle phénolique (monophénols ou polyphénols), leurs dérivés qui ont des substituants tels qu'un groupe alkyle, un groupe aryle, un groupe acyle, un groupe alcoxycarbonyle, un groupe carboxy, des esters de ceux-ci, un groupe amido ou un atome d'halogène, et les résines condensées phénol-aldéhyde

tels que des bisphénols ou des trisphénols.

Au sens de la présente invention, on entend par :

- Alkyle : un groupe aliphatique hydrocarboné saturé, linéaire ou ramifié, en $C_1$-$C_{20}$, de préférence en $C_1$-$C_{12}$, plus préférentiellement en $C_1$-$C_6$, et encore plus préférentiellement en $C_1$-$C_4$. Le terme « ramifié » signifie qu'au moins un groupe alkyle inférieur tel qu'un méthyle ou un éthyle est porté par une chaîne alkyle linéaire. A titre de groupe alkyle, on peut mentionner par exemple les groupes méthyle, éthyle, n-propyle, i-propyle, n-butyle, t-butyle et n-pentyle.
- Aryle : tout groupe fonctionnel ou substituant dérivé d'au moins un cycle aromatique ; un cycle aromatique correspond à tout groupe mono- ou polycyclique plan comportant un système $\pi$ délocalisé dans lequel chaque atome du cycle comporte une orbitale p, lesdites orbitales p se recouvrant les unes les autres ; parmi de tels groupes aryle, on peut mentionner les groupes phényle, biphényle, naphthalène et anthracène. Les groupes aryles de l'invention comprennent de préférence de 4 à 12 atomes de carbone, et de manière encore plus préférée de 5 à 6 atomes de carbone. De manière encore plus préférée, le groupe aryle de l'invention est un groupe phényle.

Ainsi, le composé accepteur d'électrons (B) peut être choisi parmi le 2,2-bis(4-hydroxy-3-méthylphényl)propane (Bisphénol C, n° CAS: 79-97-0), le 4-hexyl-1,3-dihydroxybenzène (4-hexylrésorcinol, n° CAS: 136-77-6), le 4,4'-cyclohexylidènebisphénol (BPZ, n° CAS : 843-55-0), le 4,4'-(hexafluoroisopropylidène)diphénol (Bisphénol AF, n° CAS: 1478-61-1), le 4,4'-(1-phényléthylidène)bisphénol (n° CAS: 1571-75-1), le 2,2'-dihydroxybiphényl (n° CAS : 1806-29-7), le 4,4'-éthylidènebisphénol (n° CAS: 2081-08-5), le 4,4'-(1,4-phénylènediisopropylidène)bisphénol (n° CAS: 2167-51-3), le 1,1-bis(4-hydroxy-3-méthylphényl)cyclohexane (n° CAS: 2362-14-3), le 9,9-bis(4-hydroxyphényl)fluorène (n° CAS: 3236-71-3), le 4,4'-(1,3-phénylènediisopropylidène)bisphénol (n° CAS : 13595-25-0), le 1,1,1-tris(4-hydroxyphényl)éthane (n° CAS: 27955-94-8), le 4,4'-(2-éthylhexylidène)diphénol (n° CAS: 74462-02-5), le a,a,a'-tris(4-hydroxyphényl)-1-éthyl-4-isopropylbenzène (n° CAS : 110726-28-8), le 4-(1,1,3,3-tétraméthylbutyl)phénol (n° CAS : 140-66-9), le 4-hydroxydiphényléther (n° CAS: 831-82-3), le bis(2-hydroxy-1-naphthyl)méthane (n° CAS: 1096-84-0), le 4-(méthylsulfonyl)phénol (n° CAS: 14763-60-1), le 4-hydroxyphényl-4'-isopropoxyphényl sulfone (n° CAS: 95235-30-6), le 4,4'-dihydroxybiphényl (n° CAS: 92-88-6), le 4-hydroxybiphényl (n° CAS: 92-69-3), le p-hydroxycumène (n° CAS: 99-89-8), le 2,4-dihydroxybenzophénone (n° CAS: 131-56-6), l'hydroquinone monométhyléther (MEHQ, n° CAS : 150-76-5), le 3-n-pentadécylphénol (n° CAS: 501-24-6), le 4-(2-phénylisopropyl)phénol (n° CAS: 599-64-4), le 5-chloro-2-(2,4-dichlorophénoxy)phénol (n° CAS: 3380-34-5), le N-(p-toluènesulfonyl)-N'-(3-(p-toluènesulfonyloxy)phényl)urée (n° CAS: 232938-43-1), le 2,2-bis(3,5-dibromo-4-hydroxyphényl)propane (n° CAS : 79-94-7), le 4,4'-isopropylidènediphénol (n° CAS: 80-05-7), et le 4,4'-sulfonyldiphénol, (BPS, n° CAS: 80-09-1).

De manière préférée, le composé accepteur d'électrons (B) est choisi parmi le 2,2-bis(4-hydroxy-3-méthylphényl)propane (Bisphénol C, n° CAS : 79-97-0), le 4-hexyl-1,3-dihydroxybenzène (4-hexylrésorcinol, n° CAS: 136-77-6), le 4,4'-cyclohexylidènebisphénol (BPZ, n° CAS : 843-55-0), le 4,4'-(hexafluoroisopropylidène)diphénol (Bisphénol AF, n° CAS: 1478-61-1), le 4,4'-(1-phényléthylidène)bisphénol (n° CAS: 1571-75-1), le 2,2'-dihydroxybiphényl (n° CAS : 1806-29-7), le 4,4'-(1,4-phénylènediisopropylidène)bisphénol (n° CAS : 2167-51-3), le 1,1-bis(4-hydroxy-3-méthylphényl)cyclohexane (n° CAS: 2362-14-3), le 9,9-bis(4-hydroxyphényl)fluorène (n° CAS : 3236-71-3), le 4,4'-(1,3-phénylènediisopropylidène)bisphénol (n° CAS: 13595-25-0), le 1,1,1-tris(4-hydroxyphényl)éthane (n° CAS: 27955-94-8), le 4,4'-(2-éthylhexylidène)diphénol (n° CAS: 74462-02-5), et le a,a,a'-tris(4-hydroxyphényl)-1-éthyl-4-isopropylbenzène (n° CAS : 110726-28-8).

La composition de pigment thermochrome de l'invention est préparée par dissolution des composés (A) et (B) dans le composé (C) de formule (I) de l'invention, puis agitation jusqu'à obtention d'un mélange homogène à l'aide d'un agitateur tel qu'un homo mélangeur ou un disperseur.

Les composés (A) et (B) ainsi associés avec le composé de formule (I) de l'invention peuvent être formulés sous la forme de microcapsules. Ainsi, la composition de pigment thermochrome de l'invention est encapsulée dans des microcapsules pour former des microcapsules de pigment thermochrome. De telles microcapsules de pigment thermochrome constituent un autre objet selon l'invention. Ils présentent des caractéristiques avantageuses dans la mesure où ils sont résistants aux contraintes mécaniques, insolubles et donc dispersables dans l'eau, et d'agglomération lente.

La température de fusion (ou température de décoloration T4) des microcapsules de pigment thermochrome de l'invention peut varier de 20 à 80°C, de préférence de 30 à 80°C, et encore plus préférentiellement de 40 à 70°C.

La température de cristallisation (ou température de recoloration T1) des microcapsules de pigment thermochrome de l'invention peut varier de -40 à 20°C, de préférence de -30 à 10°C, et encore plus préférentiellement de -20 à 0°C.

Les microcapsules de pigment thermochrome de l'invention présente un diamètre moyen pouvant aller de 0,5 à 30 $\mu$m, de préférence de 1 à 10 $\mu$m , et encore plus préférentiellement de 3-5 $\mu$m. Ce diamètre moyen correspond au d90 en volume et signifie que 90% en volume des microcapsules sont constitués de microcapsules ayant une taille

comprise dans l'intervalle indiquée. Ce diamètre moyen peut être déterminé par granulométrie laser en utilisant un appareil Zetasizer Nano ZS de Malvern Instruments.

Les procédés de micro-encapsulation utilisés incluent à titre non limitatif des méthodes conventionnelles, telles que :

- des procédés chimiques qui reposent sur la formation *in situ* des microcapsules enrobantes, comme par exemple par polymérisation ou polycondensation interfaciale, ces procédés étant les préférés,
- des procédés physico-chimiques, comme par exemple par séparation de phases ou coacervation, par évaporation-extraction de solvant, par gélification thermique d'émulsions (hot melt), ou
- des procédés mécaniques, comme par exemple par nébulisation/séchage (spray drying), par gélification ou congélation de gouttes, par enrobage en lit fluidisé (spray-coati ng).

Les microcapsules de pigment thermochrome de l'invention sont avantageusement à base de résine aminoplaste, et de préférence à base de résine mélamine, de résine urée ou de résine benzoguanamine.

Les microcapsules de pigment thermochrome de l'invention sont de préférence préparées par polymérisation *in situ à* partir de résine mélamine.

Un autre objet de l'invention vise une composition d'encre comprenant des microcapsules de pigment thermochrome selon l'invention.

Les microcapsules de pigment thermochrome de l'invention présentes au sein de la composition d'encre représentent de 5 à 50% en poids du poids total de la composition d'encre.

La composition d'encre de l'invention est par ailleurs majoritairement constituée d'eau. Avantageusement, l'eau représente 40 à 80% en poids du poids total de la composition d'encre.

La composition d'encre de l'invention peut également contenir un ou plusieurs co-solvants miscibles dans l'eau. Ainsi, la composition d'encre de l'invention peut contenir un solvant organique ou aqueux, de préférence un solvant aqueux.

Parmi les solvants qui peuvent être ajoutés à la composition d'encre de l'invention, on peut citer l'eau et les solvants polaires miscibles dans l'eau, comme par exemple :

- les alcools : les alcools linéaires ou ramifiés en $C_1$-$C_{15}$ tels que l'isopropanol, le butanol, l'isobutanol, le pentanol, l'alcool benzylique ; la glycérine ; la diglycérine ; la polyglycérine
- les esters tels que l'acétate d'éthyle ou l'acétate de propyle,
- les esters de carbonate tels que le carbonate de propylène ou le carbonate d'éthylène,
- les cétones telles que la méthylisobutylcétone (MIBC), l'acétone ou la cyclohexanone,
- les glycols tels que l'éthylène glycol, le diéthylène glycol, le triéthylène glycol, le propylène glycol, le polyéthylène glycol, l'éthylène glycol monométhyl éther, le 3-butylène glycol et le thiodiéthylène glycol,
- les amides tels que le diméthylacétamide ou le diméthylformamide, et
- leur mélange.

Le ou les solvants représentent de 5 à 20% en poids du poids total de la composition d'encre.

La composition d'encre de l'invention peut également contenir un ou plusieurs adjuvants spécifiques qui peuvent jouer différents rôles selon l'application finale visée. Ces applications peuvent concerner l'impression d'encre par sérigraphie, l'impression offset, l'impression par héliogravure, le revêtement par pulvérisation, le revêtement électrostatique, le revêtement par dépôt électrolytique, le revêtement au rouleau, l'impression au jet d'encre, les encres pour outils d'écriture tels que les stylos à bille, les stylos pinceaux, les marqueurs, les crayons de couleurs. La composition d'encre de l'invention peut également être ajoutée à une composition de résine thermoplastique ou thermodurcissable pour former des pièces moulées.

Parmi les adjuvants mentionnés ci-dessus, on peut citer :

- des modificateurs de rhéologie (agent rhéofluidifiant) capables de générer un effet gélifiant, tels que la gomme de xanthane ou la gomme arabique,
- des antimousses, tels que les dispersions aqueuses de polysiloxane modifiés (MOUSSEX® de Synthron),
- des régulateurs de pH, tels que l'hydroxyde de sodium, la triéthanolamine,
- des tensioactifs, tels que les polyéthers polyols (TERGITOL™ de DOW),
- des biocides, tels que les isothiazolinones (ACTICIDE® de Thor),
- des anticorrosifs, tel que la benzotriazole,
- des lubrifiants,
- des dispersants,
- des agents de coalescence,

- des agents réticulants,
- des agents mouillants,
- des plastifiants,
- des antioxydants,
- des stabilisateurs UV.

Un objet supplémentaire de l'invention concerne des instruments d'écriture comprenant une composition d'encre selon l'invention. Ces instruments sont généralement constitués d'un corps comprenant la composition d'encre de l'invention, et éventuellement un élément de frottement. L'instrument d'écriture selon l'invention est avantageusement choisi parmi les stylos à bille, les crayons, les craies, et encore plus avantageusement les stylos à bille à encre effaçable par friction. L'élément de frottement de l'instrument d'écriture est de préférence une gomme.

Les supports sur lesquels peut être appliquée la composition d'encre de l'invention sont le papier, les fibres, le cuir, le plastique, le verre, le métal, le bois, le béton.

Outre les dispositions qui précèdent, l'invention comprend encore d'autres dispositions qui ressortiront du complément de description qui suit, qui se rapporte à la synthèse de composés de formule (I) selon l'invention, à leur caractérisation, et à leur utilisation comme agent régulateur de changement de température dans des compositions de pigment thermochrome.

**EXEMPLES**

**EXEMPLE 1 :**

Les composés (6), (7), (8), (9), (10) et (11) de formules suivantes :

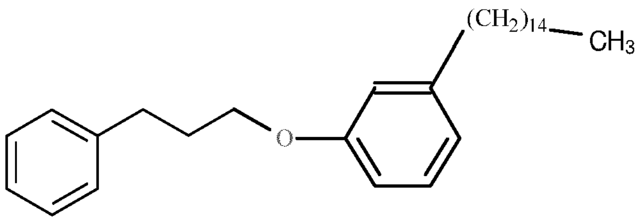

(6)

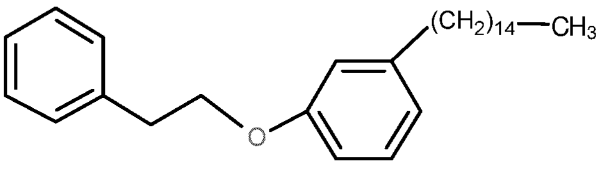

(7)

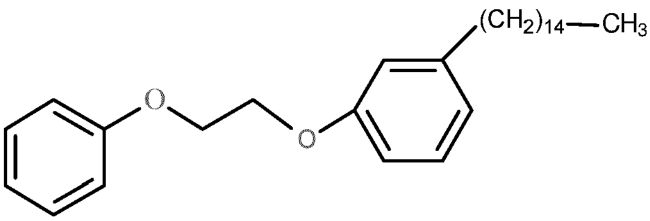

(8)

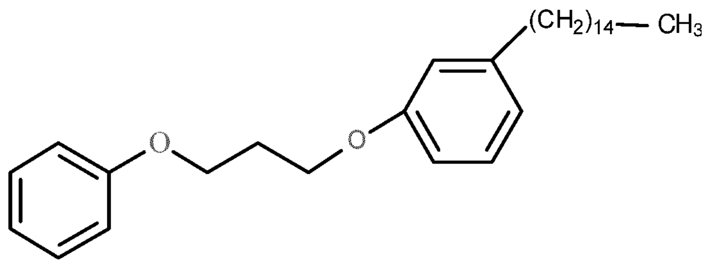

(9)

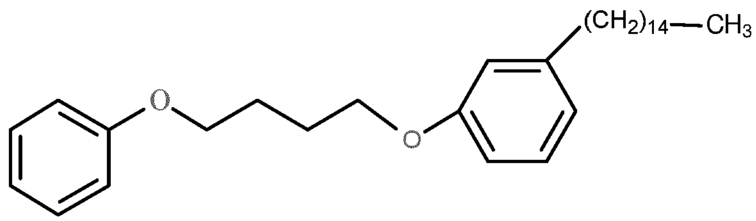

(10)

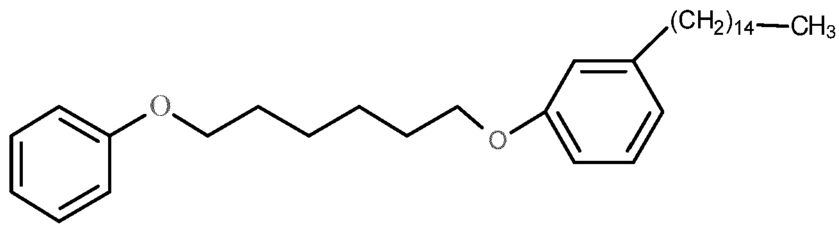

(11)

sont préparés :

- Soit par solubilisation des réactifs dans l'acétonitrile, en présence de carbonate de potassium, sous reflux (85°C) pendant 7 jours, selon le schéma de synthèse suivant :

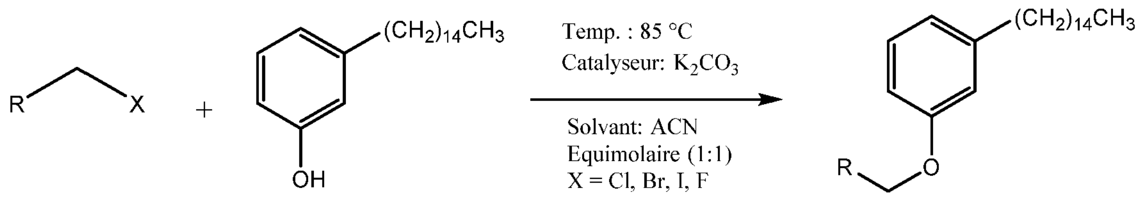


Le carbonate de potassium est ensuite éliminé par filtration.

- Soit par solubilisation des réactifs dans le *tert-butanol,* en présence d'hydroxyde de sodium, sous reflux (85°C) pendant 6 heures, selon le schéma de synthèse suivant :

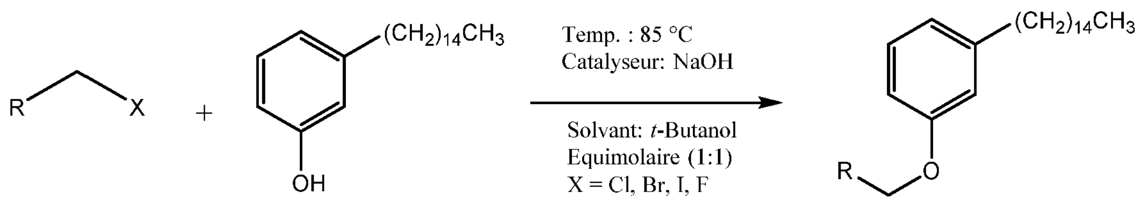


Les composés (6), (7), (8), (9), (10) et (11) ainsi synthétisés sont purifiés par recristallisation successives.

Synthèse du composé (6) :

20 g de 3-pentadécylphénol (n° CAS 501-24-6), 16 g de 1-bromo-3-phénylpropane (n° CAS 637-59-2), et 11 g de carbonate de calcium $K_2CO_3$ (n° CAS 584-08-7) sont mélangés dans 100 mL d'acétonitrile, puis chauffés à reflux à 85°C pendant 7 jours.

Le carbonate de potassium $K_2CO_3$ est éliminé par filtration. L'acétonitrile est ensuite évaporé, et le solide obtenu recristallisé dans 150 mL d'éthanol (pureté : 95%). Le milieu réactionnel est chauffé à reflux jusqu'à solubilisation complète, puis refroidi à température ambiante jusqu'à précipitation du produit. Le solide obtenu est lavé trois fois avec 50 mL d'éthanol (pureté : 95%).

L'analyse par chromatographie en phase gazeuse (GPC) du produit obtenu, réalisée à l'aide d'un appareil PerkinElmer Clarus® 680/600S, révèle que le produit est pur à 96%.

Synthèse du composé (7) :

19.7 g de 3-pentadécylphénol (n° CAS 501-24-6) sont solubilisés dans 20 mL de *tert-butanol,* puis chauffés à 40°C pour générer un mélange homogène. 3,4 g d'hydroxyde de sodium (n° CAS 1310-73-2) sont dilués dans 10 mL d'eau distillée, puis ajoutés au milieu réactionnel. Après 15 minutes d'agitation, 10 g de (2-bromoéthyl) benzène (n° CAS 103-63-9) sont ajoutés au mélange, et le milieu réactionnel chauffé à 80°C pendant 6 heures.

Le produit solide est récupéré par recristallisation et filtration, puis purifié par trois recristallisations successives dans 200 mL d'éthanol.

L'analyse par chromatographie en phase gazeuse (GPC) du produit obtenu, réalisée à l'aide d'un appareil PerkinElmer Clarus® 680/600S, révèle que le produit est pur à 96%.

Synthèse du composé (8):

20 g de 3-pentadécylphénol (n° CAS 501-24-6), 16 g de β-bromophénétole (n° CAS 589-10-6) et 11 g de carbonate de calcium $K_2CO_3$ (n° CAS 584-08-7) sont mélangés dans 100 mL d'acétonitrile, puis chauffés à reflux à 85°C pendant 7 jours.

Le carbonate de potassium $K_2CO_3$ est éliminé par filtration. L'acétonitrile est ensuite évaporé, et le solide obtenu recristallisé dans 150 mL d'éthanol (pureté : 95%). Le milieu réactionnel est chauffé à reflux jusqu'à solubilisation complète, puis refroidi à température ambiante jusqu'à précipitation du produit. Le solide obtenu est lavé trois fois avec 50 mL d'éthanol (pureté : 95%).

L'analyse par chromatographie en phase gazeuse (GPC) du produit obtenu, réalisée à l'aide d'un appareil PerkinElmer Clarus® 680/600S, révèle que le produit est pur à 99%.

Synthèse du composé (9) :

17 g de 3-pentadécylphénol (n° CAS 501-24-6) sont solubilisés dans 100 mL de *tert-butanol,* puis chauffés à 40°C pour générer un mélange homogène. 2,9 g d'hydroxyde de sodium (n° CAS 1310-73-2) sont dilués dans 10 mL d'eau distillée, puis ajoutés au milieu réactionnel. Après 15 minutes d'agitation, 10 g de bromure de 3-phénoxypropyle (n° CAS 588-63-6) sont ajoutés au mélange, et le milieu réactionnel chauffé à 80°C pendant 6 heures.

Le produit solide est récupéré par recristallisation et filtration, puis purifié par trois recristallisations successives dans 150 mL d'éthanol.

L'analyse par chromatographie en phase gazeuse (GPC) du produit obtenu, réalisée à l'aide d'un appareil PerkinElmer Clarus® 680/600S, révèle que le produit est pur à 99%.

Synthèse du composé (10):

15.8 g de 3-pentadécylphénol (n° CAS 501-24-6) sont solubilisés dans 100 mL de *tert-butanol,* puis chauffés à 40°C pour générer un mélange homogène. 2,8 g d'hydroxyde de sodium (n° CAS 1310-73-2) sont dilués dans 10 mL d'eau distillée, puis ajoutés au milieu réactionnel. Après 15 minutes d'agitation, 10 g de 4-bromobutyl phényle éther (n° CAS 1200-03-9) sont ajoutés au mélange, et le milieu réactionnel chauffé à 80°C pendant 6 heures.

Le produit solide est récupéré par recristallisation et filtration, puis purifié par trois recristallisations successives dans 150 mL d'éthanol.

L'analyse par chromatographie en phase gazeuse (GPC) du produit obtenu, réalisée à l'aide d'un appareil PerkinElmer Clarus® 680/600S, révèle que le produit est pur à 98%.

Synthèse du composé (11):

14 g de 3-pentadécylphénol (n° CAS 501-24-6) sont solubilisés dans 20 mL de *tert-butanol,* puis chauffés à 40°C pour générer un mélange homogène. 2,5 g d'hydroxyde de sodium (n° CAS 1310-73-2) sont dilués dans 10 mL d'eau distillée, puis ajoutés au milieu réactionnel. Après 15 minutes d'agitation, 10 g de bromure de 6-phénoxypropyle (n° CAS 57795-97-2) sont ajoutés au mélange, et le milieu réactionnel chauffé à 80°C pendant 8 heures.

Le produit solide est récupéré par recristallisation et filtration, puis purifié par trois recristallisations successives dans 150 mL d'éthanol.

L'analyse par chromatographie en phase gazeuse (GPC) du produit obtenu, réalisée à l'aide d'un appareil PerkinElmer Clarus® 680/600S, révèle que le produit est pur à 96%.

Les températures de fusion $T_{FUS}$ des composés (6), (7), (8), (9), (10) et (11) obtenus sont mesurées par calorimétrie différentielle à balayage (DSC) grâce à un appareil TA Instruments Q20, sur une plage de température allant de -50 à 100°C, à des vitesses de chauffage/refroidissement de +/-20°C/minute. Les températures mesurées sont indiquées dans le **Tableau 1** ci-après.

**Tableau 1 :**

| Composé de formule (I) | $T_{FUS}$ (°C) |
|---|---|
| Composé (6) | 35 |
| Composé (7) | 44 |
| Composé (8) | 71 |
| Composé (9) | 56 |
| Composé (10) | 59 |
| Composé (11) | 54 |

**EXEMPLE 2 :**

Les composés (1), (2), (3), (4), (5), (12), (13), (14), (15) et (16) de formules suivantes :

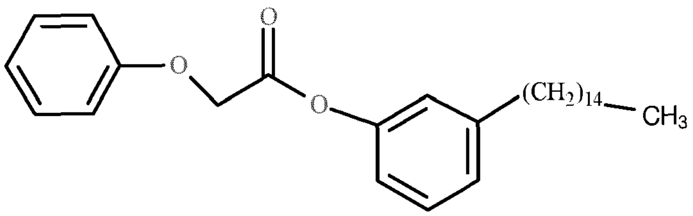

(1)

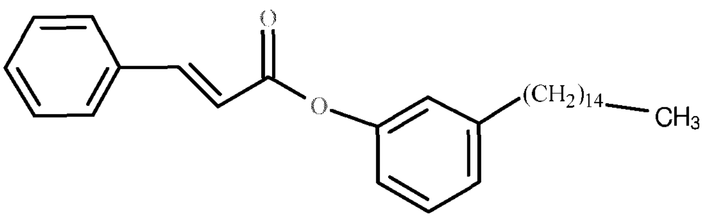

(2)

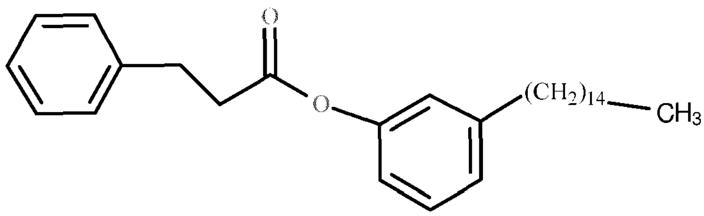

(3)

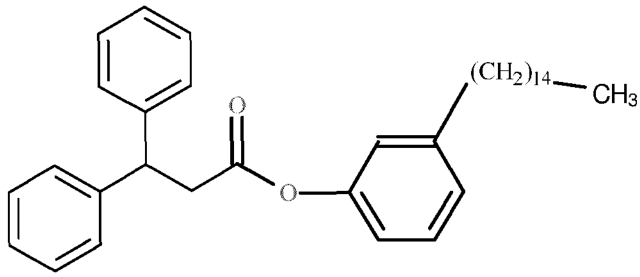

(4)

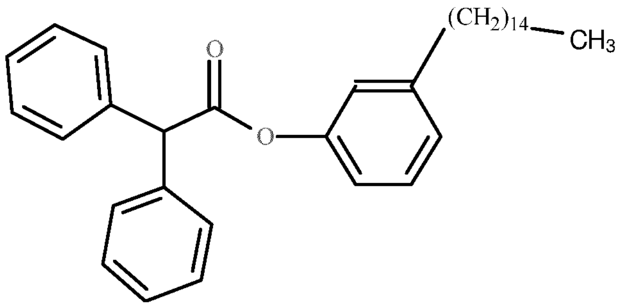

(5)

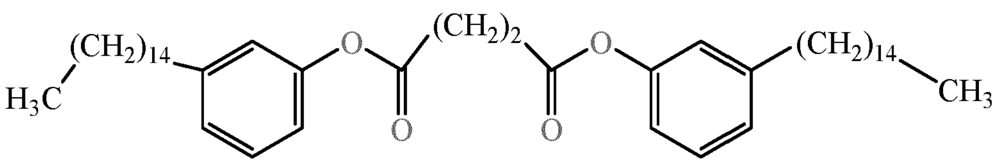

(12)

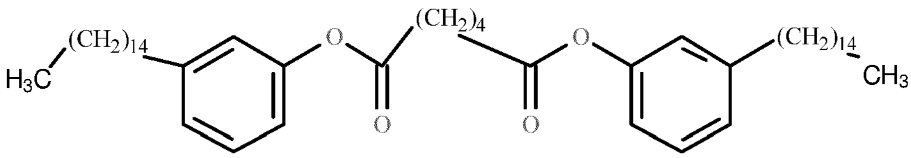

(13)

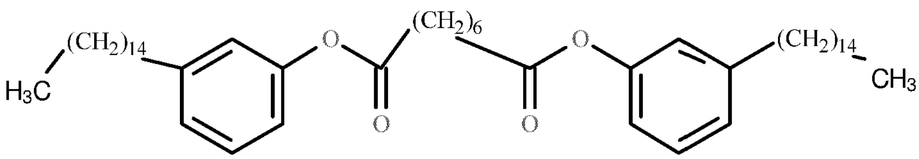

(14)

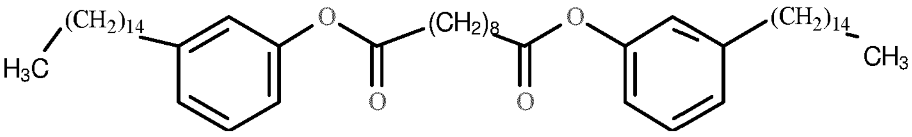

(15)

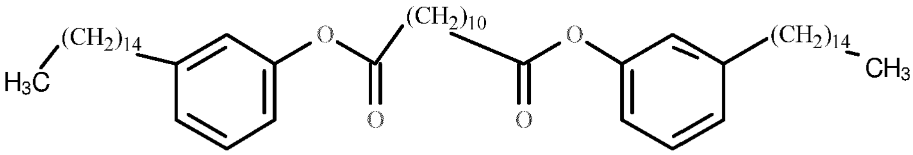

(16)

sont préparés (composé (2) est comparatif) :

- Soit à partir d'un acide carboxylique, en utilisant un alcool ayant un point de fusion inférieur à 60°C comme solvant et de l'acide paratoluènesulfonique comme catalyseur de réaction. Le mélange est chauffé à une température comprise entre 120 et 150°C, sous un léger vide, pour permettre l'élimination de l'eau et donc le déplacement de l'équilibre vers la synthèse des composés de formule (I).

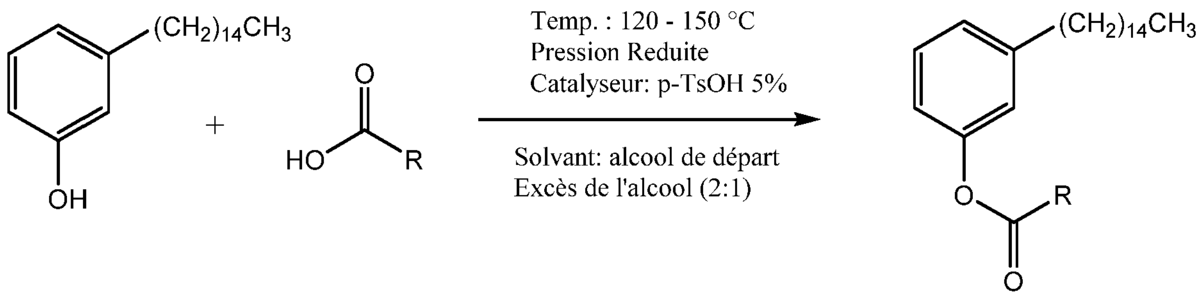


Les composés de formule (I) obtenus sont purifiés par recristallisation avec un alcool simple.

- Soit à partir d'un alcool, en le solubilisant dans du tétrahydrofurane (THF) avec de la triéthylamine comme catalyseur, à une température comprise entre 0 et 20°C. Le chlorure d'acide est ensuite ajouté lentement, au goutte-à-goutte, en 30 minutes. Le mélange est ensuite réchauffé à température ambiante (25°C) pendant 2 heures, sous agitation.

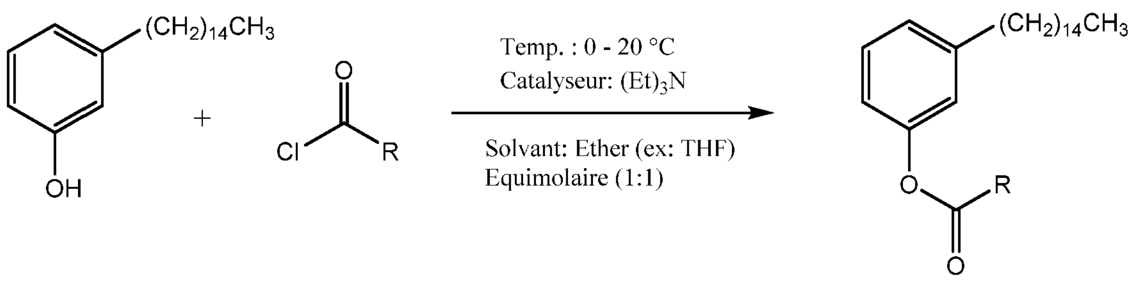


Les composés (1), (2), (3), (4), (5), (12), (13), (14), (15) et (16) obtenus sont purifiés par recristallisation.

Synthèse du composé (1) :

17,8 g de 3-pentadécylphénol (n° CAS 501-24-6) et 5,8 g de triéthylamine (n° CAS 121-44-8) sont solubilisés dans 250 mL de tétrahydrofurane (n° CAS 109-99-9). Le milieu réactionnel est maintenu à température ambiante, puis rendu inerte par ajout d'azote. 10 g de chlorure de phénoxyacétate (n° CAS 701-99-5) sont ajoutés au goutte-à-goutte pendant 15 minutes. A la fin de l'ajout, le milieu réactionnel est agité pendant 30 minutes à température ambiante.

Le milieu réactionnel est ensuite extrait avec 100 mL d'acétate d'éthyle. La phase organique est récupérée et

lavée avec trois fois 100 mL d'eau. La phase organique est ensuite séchée sur du sulfate de sodium, et le solvant évaporé.
Le produit est recristallisé deux fois par de l'isopropanol.
L'analyse par chromatographie en phase gazeuse (GPC) du produit obtenu, réalisée à l'aide d'un appareil PerkinElmer Clarus® 680/600S, révèle que le produit est pur à 95%.

Synthèse du composé comparatif (2) :

18 g de 3-pentadécylphénol (n° CAS 501-24-6) et 6,5 g de triéthylamine (n° CAS 121-44-8) sont solubilisés dans 250 mL de tétrahydrofurane (n° CAS 109-99-9). Le milieu réactionnel est maintenu à température ambiante, puis rendu inerte par ajout d'azote. 10 g de chlorure de trans-3-phénylacryloyl (n° CAS 102-92-1) dilué dans 10 mL de tétrahydrofurane sont ajoutés au goutte-à-goutte pendant 15 minutes. A la fin de l'ajout, le milieu réactionnel est agité pendant 30 minutes à température ambiante.
Le milieu réactionnel est ensuite extrait avec 150 mL d'acétate d'éthyle. La phase organique est récupérée et lavée avec trois fois 150 mL d'eau. La phase organique est ensuite séchée sur du sulfate de sodium, et le solvant évaporé.
Le produit est recristallisé deux fois par de l'éthanol.
L'analyse par chromatographie en phase gazeuse (GPC) du produit obtenu, réalisée à l'aide d'un appareil PerkinElmer Clarus® 680/600S, révèle que le produit est pur à 90%.

<u>Synthèse du composé (3)</u> :

20 g de 3-pentadécylphénol (n° CAS 501-24-6), 19,7 g d'acide 3-phénylpropionique (n° CAS 501-52-0), et 200 mg d'acide p-toluènesulfonique monohydraté (APTS) (n° CAS 6192-52-5) sont mélangés et chauffés à 140°C pendant 3 jours, sous pression réduite (400 mbar).
Le milieu réactionnel est purifié par ajout de 50 mL d'éthanol (pureté : 95%), puis chauffé à reflux jusqu'à solubilisation complète. Le milieu réactionnel est ensuite refroidi à température ambiante, jusqu'à précipitation du produit. Le solide obtenu est lavé trois fois avec 30 mL d'éthanol (pureté : 95%).
Le produit est recristallisé deux fois par de l'isopropanol.
L'analyse par chromatographie en phase gazeuse (GPC) du produit obtenu, réalisée à l'aide d'un appareil PerkinElmer Clarus® 680/600S, révèle que le produit est pur à 83%.

<u>Synthèse du composé (4)</u> :

20 g de 3-pentadécylphénol (n° CAS 501-24-6), 30 g d'acide 3,3-diphénylpropionique (n° CAS 606-83-7), et 200 mg d'acide p-toluènesulfonique monohydraté (APTS) (n° CAS 6192-52-5) sont mélangés et chauffés à 160°C pendant une journée, sous pression réduite (600 mbar).
Le milieu réactionnel est purifié par ajout de 150 mL d'éthanol (pureté : 95%), puis chauffé à reflux jusqu'à solubilisation complète. Le milieu réactionnel est ensuite refroidi à température ambiante, jusqu'à précipitation du produit. Le solide obtenu est lavé trois fois avec 30 mL d'éthanol (pureté : 95%).
L'analyse par chromatographie en phase gazeuse (GPC) du produit obtenu, réalisée à l'aide d'un appareil PerkinElmer Clarus® 680/600S, révèle que le produit est pur à 95%.

<u>Synthèse du composé (5)</u>:

13,8 g de 3-pentadécylphénol (n° CAS 501-24-6) et 5,0 g de triéthylamine (n° CAS 121-44-8) sont solubilisés dans 150 mL de tétrahydrofurane (n° CAS 109-99-9). Le milieu réactionnel est maintenu à température ambiante et est rendu inerte par ajout d'azote. 10,4 g de chlorure de diphénylacétyl (n° CAS 1871-76-7) dilués dans 15 mL de tétrahydrofurane sont ajoutés au goutte-à-goutte pendant 15 minutes. A la fin de l'ajout, le milieu réactionnel est agité pendant 30 minutes à température ambiante.
Le milieu réactionnel est ensuite extrait avec 100 mL d'acétate d'éthyle. La phase organique est récupérée et lavée avec trois fois 150 mL d'eau. La phase organique est ensuite séchée sur du sulfate de sodium, et le solvant évaporé.
Le produit est recristallisé deux fois par de l'éthanol refroidi a -20°C.
L'analyse par chromatographie en phase gazeuse (GPC) du produit obtenu, réalisée à l'aide d'un appareil PerkinElmer Clarus® 680/600S, révèle que le produit est pur à 86%.

<u>Synthèse du composé (12)</u> :

38,9 g de 3-pentadécylphénol (n° CAS 501-24-6) et 6.8 g de triéthylamine (n° CAS 121-44-8) sont solubilisés dans 250 mL de tétrahydrofurane (n° CAS 109-99-9). Le milieu réactionnel est maintenu à température ambiante et est

rendu inerte par ajout d'azote. 10 g de chlorure d'acide succinique (n° CAS 543-20-4) sont ajoutés au goutte-à-goutte pendant 15 minutes. A la fin de l'ajout, le milieu réactionnel est agité pendant 30 minutes à température ambiante.

Le produit obtenu est extrait avec 100 mL d'acétate d'éthyle. La phase organique est récupérée et lavée avec trois fois 150 mL d'eau. La phase organique est ensuite séchée sur du sulfate de sodium, et le solvant évaporé.

Le produit est recristallisé deux fois par de l'isopropanol.

L'analyse par chromatographie en phase gazeuse (GPC) du produit obtenu, réalisée à l'aide d'un appareil PerkinElmer Clarus® 680/600S, révèle que le produit est pur à 94%.

Synthèse du composé (13):

33,1 g de 3-pentadécylphénol (n° CAS 501-24-6) et 8,1 g de triéthylamine (n° CAS 121-44-8) sont solubilisés dans 250 mL de tétrahydrofurane (n° CAS 109-99-9). Le milieu réactionnel est maintenu à température ambiante et est rendu inerte par ajout d'azote. 10 g de chlorure d'acide adipique (n° CAS 111-50-2) sont ajoutés au goutte-à-goutte pendant 15 minutes. A la fin de l'ajout, le milieu réactionnel est agité pendant 30 minutes à température ambiante.

Le produit obtenu est extrait avec 100 mL d'acétate d'éthyle. La phase organique est récupérée et lavée avec trois fois 150 mL d'eau. La phase organique est ensuite séchée sur du sulfate de sodium, et le solvant évaporé.

Le produit est recristallisé deux fois par de l'isopropanol.

L'analyse par chromatographie en phase gazeuse (GPC) du produit obtenu, réalisée à l'aide d'un appareil PerkinElmer Clarus® 680/600S, révèle que le produit est pur à 87%.

Synthèse du composé (14) :

15,9 g de 3-pentadécylphénol (n° CAS 501-24-6) et 5,8 g de triéthylamine (n° CAS 121-44-8) sont solubilisés dans 250 mL de tétrahydrofurane (n° CAS 109-99-9). Le milieu réactionnel est maintenu à température ambiante et est rendu inerte par ajout d'azote. 10 g de chlorure d'acide subérique (n° CAS 10027-07-3) sont ajoutés au goutte-à-goutte pendant 15 minutes. A la fin de l'ajout, le milieu réactionnel est agité pendant 30 minutes à température ambiante.

Le produit obtenu est extrait avec 100 mL d'acétate d'éthyle. La phase organique est récupérée et lavée avec trois fois 150 mL d'eau. La phase organique est ensuite séchée sur du sulfate de sodium, et le solvant évaporé.

Le produit est recristallisé deux fois par de l'isopropanol.

L'analyse par chromatographie en phase gazeuse (GPC) du produit obtenu, réalisée à l'aide d'un appareil PerkinElmer Clarus® 680/600S, révèle que le produit est pur à 96%.

Synthèse du composé (15) :

20 g de 3-pentadécylphénol (n° CAS 501-24-6) et 7,2 g de triéthylamine (n° CAS 121-44-8) sont solubilisés dans 250 mL de tétrahydrofurane (n° CAS 109-99-9). Le milieu réactionnel est refroidi à 5°C, et maintenu à cette température dans un bain de glace. Le milieu réactionnel est rendu inerte par ajout d'azote. 7,9 g de chlorure de sébacoyle (n° CAS 111-19-3) sont ajoutés au goutte-à-goutte pendant 15 minutes. Le milieu réactionnel est ensuite agité pendant 2 heures à température ambiante.

Le produit obtenu est extrait avec 200 mL d'acétate d'éthyle. La phase organique est récupérée et lavée avec trois fois 100 mL d'eau. La phase organique est ensuite séchée sur du sulfate de sodium, et le solvant évaporé.

L'analyse par chromatographie en phase gazeuse (GPC) du produit obtenu, réalisée à l'aide d'un appareil PerkinElmer Clarus® 680/600S, révèle que le produit est pur à 90%.

Synthèse du composé (16) :

22,7 g de 3-pentadécylphénol (n° CAS 501-24-6) et 8,3 g de triéthylamine (n° CAS 121-44-8) sont solubilisés dans 250 mL de tétrahydrofurane (n° CAS 109-99-9). Le milieu réactionnel est maintenu à température ambiante et est rendu inerte par ajout d'azote. 10 g de chlorure d'acide dodecanoïque (n° CAS 4834-98-4) sont ajoutés au goutte-à-goutte pendant 15 minutes. A la fin de l'ajout, le milieu réactionnel est agité pendant 30 minutes à température ambiante.

Le produit obtenu est extrait avec 100 mL d'acétate d'éthyle. La phase organique est récupérée et lavée avec trois fois 150 mL d'eau. La phase organique est ensuite séchée sur du sulfate de sodium, et le solvant évaporé.

Le produit est recristallisé deux fois par de l'isopropanol.

L'analyse par chromatographie en phase gazeuse (GPC) du produit obtenu, réalisée à l'aide d'un appareil PerkinElmer Clarus® 680/600S, révèle que le produit est pur à 94%.

Les températures de fusion des composés (1), (2), (3), (4), (5), (12), (13), (14), (15) et (16) obtenus sont mesurées par calorimétrie différentielle à balayage (DSC) grâce à un appareil TA Instruments Q20, sur une plage de température allant de -50 à 100°C, à des vitesses de chauffage/refroidissement de +/-20°C/minute. Les températures

mesurées sont indiquées dans le **Tableau 2** ci-après.

**Tableau 2 :**

| Composé de formule (I) | $T_{FUS}$ (°C) |
|---|---|
| Composé (1) | 41 |
| Composé (2) | 53 |
| Composé (3) | 43 |
| Composé (4) | 58 |
| Composé (5) | 40 |
| Composé (12) | 53 |
| Composé (13) | 54 |
| Composé (14) | 55 |
| Composé (15) | 55 |
| Composé (16) | 58 |

**EXEMPLE 3 :**

Préparation d'une composition de pigment thermochrome :
Une composition de pigment thermochrome est préparée en mélangeant 2,2 parts en poids de 3-(4-diéthylamino-2-éthoxyphényl)-3-(1-éthyl-2-méthylindol-3-yl)-4-azaphthalide (composé (A), n° CAS: 69898-40-4), 2,2 parts en poids de 4,4'-(hexafluoroisopropylidène)diphénol (composé (B1), n° CAS: 1478-61-1), 2,2 parts en poids de 2,2-bis(4-hydroxy-3-methylphényl)propane (composé (B2), n° CAS : 79-97-0), et 93,4 parts en poids du composé (4) préalablement synthétisé (composé (C)). Le mélange obtenu est chauffé, sous agitation, à une température de 110°C pendant 45 minutes, jusqu'à solubilisation complète des composés (A), (B1) et (B2) dans le composé (C).
Préparation de microcapsules de pigment thermochrome :
7,5 parts en poids d'une solution aqueuse d'un copolymère d'anhydride maléique et de méthylvinyléther (solution à 33% en poids de copolymère) sont neutralisés avec 9,2 parts en poids d'une solution aqueuse d'hydroxyde de sodium (solution à 1,0 M) à pH = 4. Cette solution est diluée avec 41,0 parts en poids d'eau, et le mélange émulsionné avec un homogénéiseur à une vitesse d'au moins 15 $m.s^{-1}$. 25,6 parts en poids de la composition de pigment thermochrome précédemment préparée sont ajoutés, et l'émulsion obtenue est maintenue à une température de 85°C pendant 30 minutes. 16,7 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.
Un slurry constitué de microcapsules de pigment thermochrome dispersées dans un solvant aqueux est obtenu, les microcapsules présentant un diamètre d90 de 4,2 $\mu$m , déterminé à l'aide d'un système Zetasizer Nano ZS de Malvern Instruments avec une illumination à 632 nm.
Les microcapsules de pigment thermochrome obtenues ont la propriété de changer de couleur du bleu à l'incolore au-dessus de 58°C avec un effet d'hystérésis de la couleur.

**EXEMPLE 4 :**

Préparation d'une composition de pigment thermochrome :
Une composition de pigment thermochrome est préparée en mélangeant 3 parts en poids de 3-(4-diéthylamino-2-éthoxyphényl)-3-(1-éthyl-2-méthylindol-3-yl)-4-azaphthalide (composé (A), n° CAS: 69898-40-4), 2,5 parts en poids de 4,4'-(hexafluoroisopropylidène)diphénol (composé (B1) n° CAS: 1478-61-1), 2,5 parts en poids de 2,2-bis(4-hydroxy-3-méthylphényl)propane (composé (B2), n° CAS : 79-97-0), et 92 parts en poids du composé (8) préalablement synthétisé (composé (C)).
Le mélange obtenu est chauffé, sous agitation, à une température de 110°C pendant 1 heure, jusqu'à solubilisation complète des composés (A), (B1) et (B2) dans le composé (C).
Préparation de microcapsules de pigment thermochrome :
7,5 parts en poids d'une solution aqueuse d'un copolymère d'anhydride maléique et de méthylvinyléther (solution à 33% en poids de copolymère) sont neutralisés avec 8,7 parts en poids d'une solution aqueuse d'hydroxyde de sodium (solution à 1,0 M) à pH = 4. Cette solution est diluée avec 42,6 parts en poids d'eau, et le mélange émulsionné avec un homogénéiseur à une vitesse d'au moins 15 $m.s^{-1}$. 25,2 parts en poids de la composition de pigment thermochrome précédemment préparée sont ajoutés, et l'émulsion obtenue est maintenue à une température de 90°C pendant 30 minutes.

16,0 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 4,6 μm, déterminé à l'aide d'un système Zetasizer Nano ZS de Malvern Instruments avec une illumination à 632 nm.

Les microcapsules de pigment thermochrome obtenues ont la propriété de changer de couleur du bleu à l'incolore au-dessus de 71°C avec un effet d'hystérésis de la couleur.

**EXEMPLE 5 :**

Préparation d'une composition de pigment thermochrome :
Une composition de pigment thermochrome est préparée en mélangeant 2,2 parts en poids de 3-(4-diéthylamino-2-éthoxyphényl)-3-(1-éthyl-2-méthylindol-3-yl)-4-azaphthalide (composé (A), n° CAS: 69898-40-4), 2,2 parts en poids de 4,4'-(hexafluoroisopropylidène)diphénol (composé (B1), n° CAS: 1478-61-1), 2,2 parts en poids de 2,2-bis(4-hydroxy-3-méthylphényl)propane (composé (B2), n° CAS : 79-97-0), et 93,4 parts en poids du composé (15) préalablement synthétisé (composé (C).

Le mélange obtenu est chauffé, sous agitation, à une température de 110°C pendant 30 minutes, jusqu'à solubilisation complète des composés (A), (B1) et (B2) dans le composé (C).

Préparation d'un pigment thermochrome microencapsulé :
7,6 parts en poids d'une solution aqueuse d'un copolymère d'anhydride maléique et de méthylvinyléther (solution à 33% en poids de copolymère) sont neutralisés avec 9,4 parts en poids d'une solution aqueuse d'hydroxyde de sodium (solution à 1,0 M) à pH = 4. Cette solution est diluée avec 39,6 parts en poids d'eau, et le mélange émulsionné avec un homogénéiseur à une vitesse d'au moins 15 $m.s^{-1}$. 26,7 parts en poids de la composition de pigment thermochrome précédemment préparée sont ajoutés, et l'émulsion obtenue est maintenue à une température de 80°C pendant 30 minutes. 16,7 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 3,7 μm, déterminé à l'aide d'un système Zetasizer Nano ZS de Malvern Instruments avec une illumination à 632 nm.

Les microcapsules de pigment thermochrome obtenues ont la propriété de changer de couleur du bleu à l'incolore au-dessus de 55°C avec un effet d'hystérésis de la couleur.

Préparation d'une composition d'encre :
10,8 parts en poids de glycérine (co-solvant) sont chauffés à une température de 30°C, sous agitation avec une pâle. 0,2 parts en poids de benzotriazole (anticorrosif) et 0,2 parts en poids d'une solution aqueuse comprenant 2,5% en poids de 1,2-benzisothiazolin-3-one et 2,5% en poids de 2-méthyl-4-isothiazolin-3-one (biocide), 0,5 parts en poids d'une dispersion aqueuse d'un copolymère polysiloxane (dispersion aqueuse à 50% en poids de polymère) (antimousse), et 0,5 parts en poids d'un polyéther polyol (tensioactif), sont ensuite ajoutés. Le mélange est agité jusqu'à solubilisation complète des additifs. 0,5 part en poids de gomme de xanthane (modificateur de rhéologie) est ajouté lentement pendant 15 minutes. Après dispersion du modificateur de rhéologie, 26,8 parts en poids d'eau distillé sont ajoutés. La composition d'encre obtenue est maintenue sous agitation pendant 3 heures, puis 60 parts en poids d'une dispersion aqueuse de microcapsules de pigment thermochrome préparée ci-dessus (dispersion aqueuse à 30% en poids de microcapsules de pigment thermochrome) sont ajoutés. Le pH de la composition d'encre est ajusté à pH = 8 avec 0,5 part en poids de triéthanolamine. L'encre bleue est ensuite dispersée avec un disperseur à une vitesse d'au moins 15 $m.s^{-1}$ pendant 30 minutes. La composition d'encre est dégazée sous pression réduite avant injection dans des cartouches d'encre.

**EXEMPLE 6 :**

Préparation d'une composition de pigment thermochrome :
Une composition de pigment thermochrome est préparée en mélangeant 2,3 parts en poids de 3-(4-diéthylamino-2-éthoxyphényl)-3-(1-éthyl-2-méthylindol-3-yl)-4-azaphthalide (composé (A), n° CAS: 69898-40-4), 1,9 parts en poids de 4,4'-(hexafluoroisopropylidène)diphénol (composé (B1), n° CAS : 1478-61-1), 1,9 parts en poids de 2,2-bis(4-hydroxy-3-methylphényl)propane (composé (B2), n° CAS : 79-97-0), et 93,9 parts en poids du composé (2) préalablement synthétisé (composé (C)). Le mélange obtenu est chauffé, sous agitation, à une température de 110°C pendant 45 minutes, jusqu'à solubilisation complète des composés (A), (B1) et (B2) dans le composé (C).

Préparation de microcapsules de pigment thermochrome :
9,3 parts en poids d'une solution aqueuse d'un copolymère d'anhydride maléique et de méthylvinyléther (solution à 27%

en poids de copolymère) sont neutralisés avec 16,3 parts en poids d'une solution aqueuse d'hydroxyde de sodium (solution à 1,0 M) à pH = 4,5. Cette solution est diluée avec 27,6 parts en poids d'eau, et le mélange émulsionné avec un homogénéiseur à une vitesse d'au moins 15 $m.s^{-1}$. 27,0 parts en poids de la composition de pigment thermochrome précédemment préparée sont ajoutés, et l'émulsion obtenue est maintenue à une température de 85°C pendant 30 minutes. 19,8 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 2,8 µm, déterminé à l'aide d'un système Zetasizer Nano ZS de Malvern Instruments avec une illumination à 632 nm.

Les microcapsules de pigment thermochrome obtenues ont la propriété de changer de couleur du bleu à l'incolore au-dessus de 54°C avec un effet d'hystérésis de la couleur.

Préparation d'une composition d'encre :

10,5 parts en poids de glycérine (co-solvant) sont chauffés à une température de 30°C, sous agitation avec une pâle. 0,2 parts en poids de benzotriazole (anticorrosif) et 0,2 parts en poids d'une solution aqueuse comprenant 2,5% en poids de 1,2-benzisothiazolin-3-one et 2,5% en poids de 2-méthyl-4-isothiazolin-3-one (biocide), 0,5 parts en poids d'une dispersion aqueuse d'un copolymère polysiloxane (dispersion aqueuse à 50% en poids de polymère) (antimousse), et 0,8 parts en poids d'un diester sulfosuccinate (tensioactif), sont ensuite ajoutés. Le mélange est agité jusqu'à solubilisation complète des additifs. 0,5 part en poids de gomme de xanthane (modificateur de rhéologie) est ajouté lentement pendant 15 minutes. Après dispersion du modificateur de rhéologie, 26,8 parts en poids d'eau distillée sont ajoutés. La composition d'encre obtenue est maintenue sous agitation pendant 3 heures, puis 60 parts en poids d'une dispersion aqueuse de microcapsules de pigment thermochrome préparée ci-dessus (dispersion aqueuse à 30% en poids de microcapsules de pigment thermochrome) sont ajoutés. Le pH de la composition d'encre est ajusté à pH = 8 avec 0,5 part en poids de triéthanolamine. L'encre bleue est ensuite dispersée avec un disperseur à une vitesse d'au moins 15 $m.s^{-1}$ pendant 30 minutes. La composition d'encre est dégazée sous pression réduite avant injection dans des cartouches.

**EXEMPLE 7 :**

Préparation d'une composition de pigment thermochrome :

Une composition de pigment thermochrome est préparée en mélangeant 2,2 parts en poids de 3-(4-diéthylamino-2-éthoxyphényl)-3-(1-éthyl-2-méthylindol-3-yl)-4-azaphthalide (composé (A), n° CAS: 69898-40-4), 2,0 parts en poids de 4,4'-(hexafluoroisopropylidène)diphénol (composé (B1), n° CAS: 1478-61-1), 2,0 parts en poids de 2,2-bis(4-hydroxy-3-methylphényl)propane (composé (B2), n° CAS : 79-97-0), et 93,8 parts en poids du composé (9) préalablement synthétisé (composé (C)). Le mélange obtenu est chauffé, sous agitation, à une température de 110°C pendant 45 minutes, jusqu'à solubilisation complète des composés (A), (B1) et (B2) dans le composé (C).

Préparation de microcapsules de pigment thermochrome :

9,0 parts en poids d'une solution aqueuse d'un copolymère d'anhydride maléique et de méthylvinyléther (solution à 27% en poids de copolymère) sont neutralisés avec 15,7 parts en poids d'une solution aqueuse d'hydroxyde de sodium (solution à 1,0 M) à pH = 4,5. Cette solution est diluée avec 28,1 parts en poids d'eau, et le mélange émulsionné avec un homogénéiseur à une vitesse d'au moins 15 $m.s^{-1}$. 28,1 parts en poids de la composition de pigment thermochrome précédemment préparée sont ajoutés, et l'émulsion obtenue est maintenue à une température de 85°C pendant 30 minutes. 19,1 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 4,2 µm, déterminé à l'aide d'un système Zetasizer Nano ZS de Malvern Instruments avec une illumination à 632 nm.

Les microcapsules de pigment thermochrome obtenues ont la propriété de changer de couleur du bleu à l'incolore au-dessus de 56°C avec un effet d'hystérésis de la couleur.

**EXEMPLE 8 :**

Préparation d'une composition de pigment thermochrome :

Une composition de pigment thermochrome est préparée en mélangeant 2,2 parts en poids de 3-(4-diéthylamino-2-éthoxyphényl)-3-(1-éthyl-2-méthylindol-3-yl)-4-azaphthalide (composé (A), n° CAS: 69898-40-4), 2,0 parts en poids de 4,4'-(hexafluoroisopropylidène)diphénol (composé (B1), n° CAS : 1478-61-1), 2,0 parts en poids de 2,2-bis(4-hydroxy-3-methylphényl)propane (composé (B2), n° CAS : 79-97-0), et 93,8 parts en poids du composé (14) préalablement synthétisé (composé (C)). Le mélange obtenu est chauffé, sous agitation, à une température de 110°C pendant 45

minutes, jusqu'à solubilisation complète des composés (A), (B1) et (B2) dans le composé (C).

Préparation de microcapsules de pigment thermochrome :

9,4 parts en poids d'une solution aqueuse d'un copolymère d'anhydride maléique et de méthylvinyléther (solution à 27% en poids de copolymère) sont neutralisés avec 16,2 parts en poids d'une solution aqueuse d'hydroxyde de sodium (solution à 1,0 M) à pH = 4,5. Cette solution est diluée avec 27,5 parts en poids d'eau, et le mélange émulsionné avec un homogénéiseur à une vitesse d'au moins 15 $m.s^{-1}$. 27,3 parts en poids de la composition de pigment thermochrome précédemment préparée sont ajoutés, et l'émulsion obtenue est maintenue à une température de 85°C pendant 30 minutes. 19,6 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 3,2 μm, déterminé à l'aide d'un système Zetasizer Nano ZS de Malvern Instruments avec une illumination à 632 nm.

Les microcapsules de pigment thermochrome obtenues ont la propriété de changer de couleur du bleu à l'incolore au-dessus de 55°C avec un effet d'hystérésis de la couleur.

Préparation d'une composition encre :

10,3 parts en poids de glycérine (co-solvant) sont chauffés à une température de 30°C, sous agitation avec une pâle. 0,2 parts en poids de benzotriazole (anticorrosif) et 0,2 parts en poids d'une solution aqueuse comprenant 2,5% en poids de 1,2-benzisothiazolin-3-one et 2,5% en poids de 2-méthyl-4-isothiazolin-3-one (biocide), 0,5 parts en poids d'une dispersion aqueuse d'un copolymère polysiloxane (dispersion aqueuse à 50% en poids de polymère) (antimousse), et 0,5 parts en poids d'un polyéther polyol (tensioactif), sont ensuite ajoutés. Le mélange est agité jusqu'à solubilisation complète des additifs. 0,5 part en poids de gomme de xanthane (modificateur de rhéologie) est ajouté lentement pendant 15 minutes. Après dispersion du modificateur de rhéologie, 26,8 parts en poids d'eau distillé sont ajoutés. La composition d'encre obtenue est maintenue sous agitation pendant 3 heures, puis 60 parts en poids d'une dispersion aqueuse de microcapsules de pigment thermochrome préparée ci-dessus (dispersion aqueuse à 30% en poids de microcapsules de pigment thermochrome) sont ajoutés. Le pH de la composition d'encre est ajusté à pH = 8 avec 1 part en poids de NaOH. L'encre bleue est ensuite dispersée avec un disperseur à une vitesse d'au moins 15 $m.s^{-1}$ pendant 30 minutes. La composition d'encre est dégazée sous pression réduite avant injection dans des cartouches d'encre.

**EXEMPLE 9 :**

Préparation d'une composition de pigment thermochrome :

Une composition de pigment thermochrome est préparée en mélangeant 2,2 parts en poids de 3-(4-diéthylamino-2-éthoxyphényl)-3-(1-éthyl-2-méthylindol-3-yl)-4-azaphthalide (composé (A), n° CAS: 69898-40-4), 2,0 parts en poids de 4,4'-(hexafluoroisopropylidène)diphénol (composé (B1), n° CAS : 1478-61-1), 2,0 parts en poids de 2,2-bis(4-hydroxy-3-methylphényl)propane (composé (B2), n° CAS : 79-97-0), et 93,8 parts en poids du composé (16) préalablement synthétisé (composé (C)). Le mélange obtenu est chauffé, sous agitation, à une température de 110°C pendant 45 minutes, jusqu'à solubilisation complète des composés (A), (B1) et (B2) dans le composé (C).

Préparation de microcapsules de pigment thermochrome :

9,3 parts en poids d'une solution aqueuse d'un copolymère d'anhydride maléique et de méthylvinyléther (solution à 27% en poids de copolymère) sont neutralisés avec 16,0 parts en poids d'une solution aqueuse d'hydroxyde de sodium (solution à 1,0 M) à pH = 4,5. Cette solution est diluée avec 27,7 parts en poids d'eau, et le mélange émulsionné avec un homogénéiseur à une vitesse d'au moins 15 $m.s^{-1}$. 27,1 parts en poids de la composition de pigment thermochrome précédemment préparée sont ajoutés, et l'émulsion obtenue est maintenue à une température de 85°C pendant 30 minutes. 19,9 parts en poids d'un pré-polymère mélamine-formaldéhyde (solution aqueuse à 50% en poids de pré-polymère) sont ensuite ajoutés au mélange au goutte-à-goutte. Le milieu réactionnel est alors chauffé à une température de 90°C, et mélangé à une vitesse d'au moins 15 $m.s^{-1}$ pendant 4 heures.

Un slurry constitué de microcapsules de pigment thermochrome dispersées dans un solvant aqueux est obtenu, les microcapsules présentant un diamètre d90 de 3,1 μm, déterminé à l'aide d'un système Zetasizer Nano ZS de Malvern Instruments avec une illumination à 632 nm.

Les microcapsules de pigment thermochrome obtenues ont la propriété de changer de couleur du bleu à l'incolore au-dessus de 58°C avec un effet d'hystérésis de la couleur.

**Détermination des températures de décoloration et de recoloration des microcapsules de pigments thermochromes préparées aux Exemples 3, 4, 5, 6, 7, 8, et 9 :**

Les températures de transition des microcapsules de pigments thermochromes obtenues sont mesurées par calorimétrie différentielle à balayage (DSC) grâce à un appareil TA Instruments Q20, sur une plage de température

allant de -50 à 100°C, à des vitesses de chauffage/refroidissement de +/-20°C/minute. Les températures mesurées sont indiquées dans le **Tableau 3** ci-après.

**Tableau** 3 : Températures de transition des microcapsules de pigments thermochromes préparés aux Exemples 3, 4, 5, 6, 7, 8 et 9

| | Changement de couleur coloré ↔ incolore | T1 (°C) | T2 (°C) | T3 (°C) | T4 (°C) | $T_H$ (°C) | $T_G$ (°C) | ΔH |
|---|---|---|---|---|---|---|---|---|
| Microcapsules de pigment thermochrome comprenant le composé (4) (exemple 3) | bleue ↔ incolore | -20 | -10 | 48 | 58 | -15 | 53 | 68 |
| Microcapsules de pigment thermochrome comprenant le composé (8) (exemple 4) | bleue ↔ incolore | 0 | 10 | 55 | 71 | 5 | 63 | 58 |
| Microcapsules de pigment thermochrome comprenant le composé (15) (exemple 5) | bleue ↔ incolore | -5 | 5 | 40 | 55 | 0 | 48 | 48 |
| Microcapsules de pigment thermochrome comprenant le composé (2) (exemple 6) | bleue ↔ incolore | -8 | -5 | 40 | 54 | -7 | 47 | 54 |
| Microcapsules de pigment thermochrome comprenant le composé (9) (exemple 7) | bleue ↔ incolore | -7 | 18 | 38 | 56 | 6 | 47 | 41 |
| Microcapsules de pigment thermochrome Comprenant le composé (14) (exemple 8) | bleue ↔ incolore | 5 | 10 | 35 | 55 | 8 | 45 | 37 |
| Microcapsules de pigment thermochrome Comprenant le composé (16) (exemple 9) | bleue ↔ incolore | 8 | 12 | 36 | 58 | 10 | 47 | 37 |

Les températures de transition mesurées sont les suivantes :

T1 : température de recoloration complète,
T2 : température de recoloration partielle,
T3 : température de décoloration partielle,
T4 : température de décoloration complète,
$T_H = \frac{T1+T2}{2}$,
$T_G = \frac{T3+T4}{2}$,
ΔH = plage d'hystérésis = $T_G$ - $T_H$.

## Revendications

**1.** Composé répondant à la formule (I) suivante :

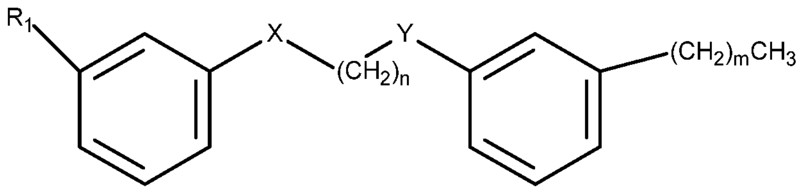

(I)

dans laquelle :

- X représente $CHR_2$, O ou OCO,
- Y représente O ou COO,
- $R_1$ représente H ou $(CH_2)_pCH_3$,
- $R_2$ représente un groupe phényle ou H,
- m = 12-18,
- n = 0-14,
- p = 12-18, et

à la condition que lorsque n = 0, X représente $CHR_2$.

2. Composé selon la revendication 1 répondant à la formule ($I_a$) suivante :

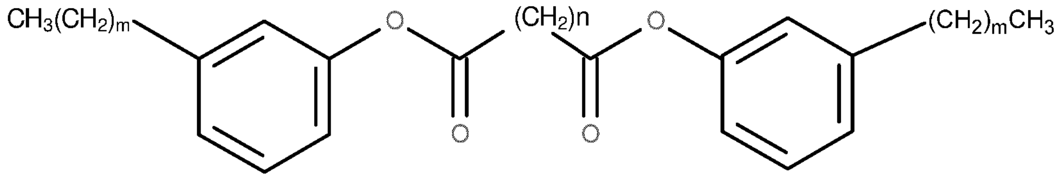

($I_a$)

dans laquelle :

- m = 12-18, et
- n = 1-14.

3. Composé selon la revendication 1 répondant à la formule ($I_b$) suivante :

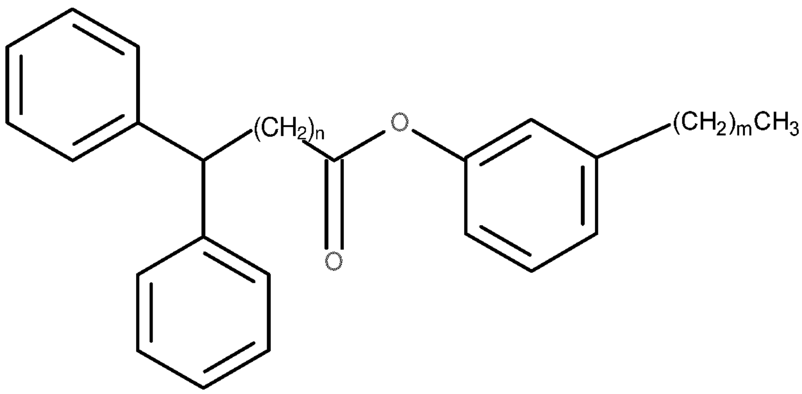

($I_b$)

dans laquelle :

- m = 12-18, et
- n = 0-14.

**4.** Composé selon la revendication 1 répondant à la formule ($I_c$) suivante :

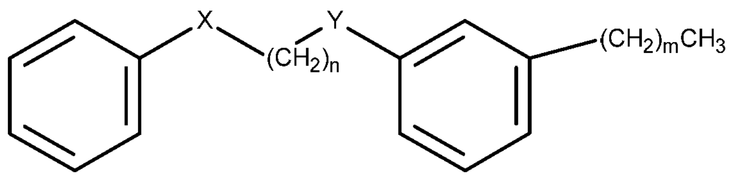

($I_c$)

dans laquelle :

- X représente $CH_2$ ou O,
- Y représente O ou COO,
- m = 12-18, et
- n = 1-14.

**5.** Composé selon l'une des revendications 1 à 4 choisi parmi un des composés suivants :

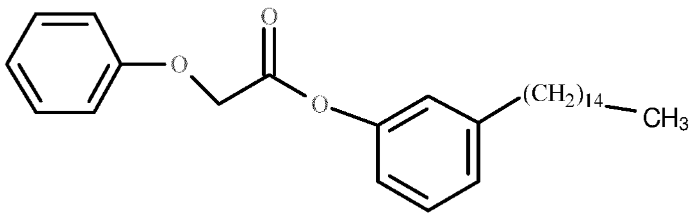

(1)

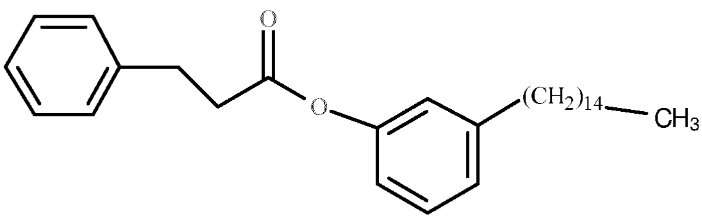

(3)

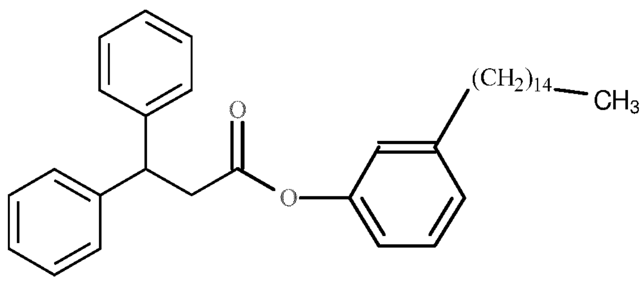

(4)

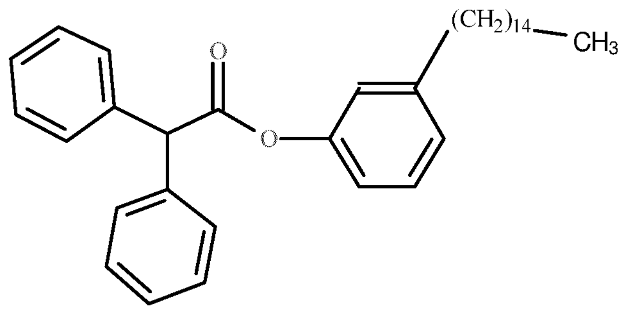

(5)

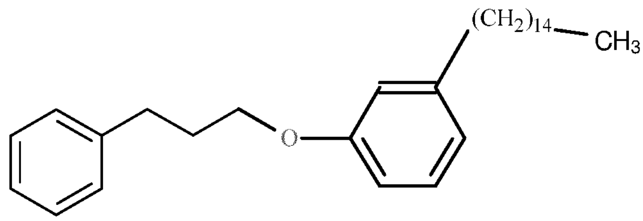

(6)

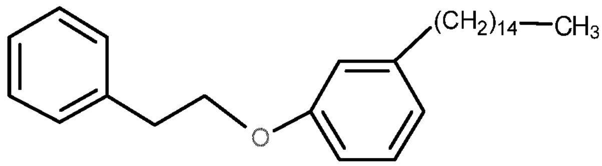

(7)

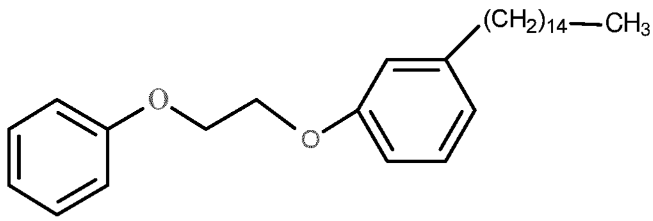

(8)

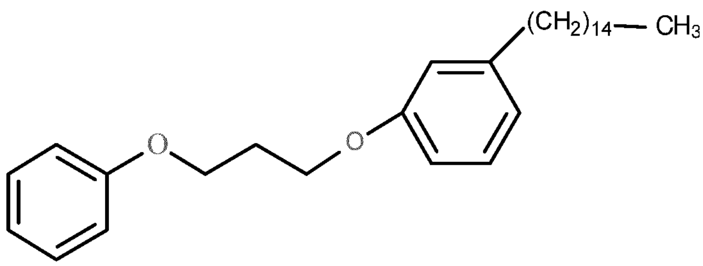

(9)

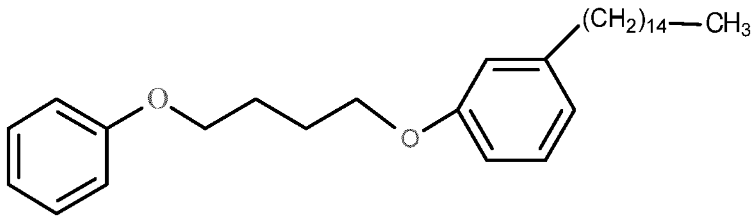

(10)

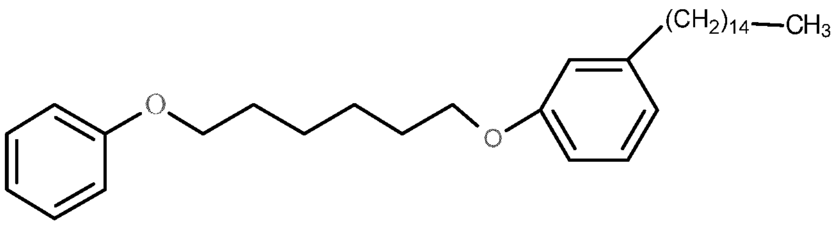

(11)

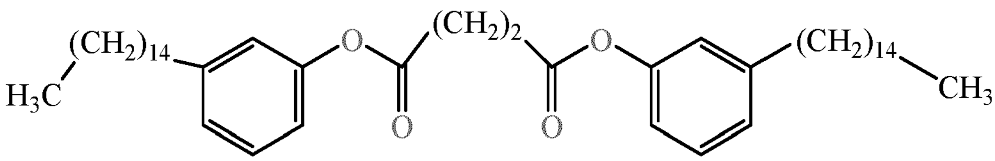

(12)

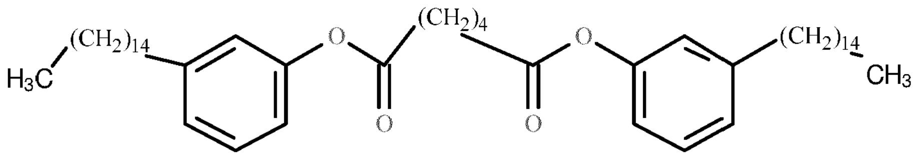

(13)

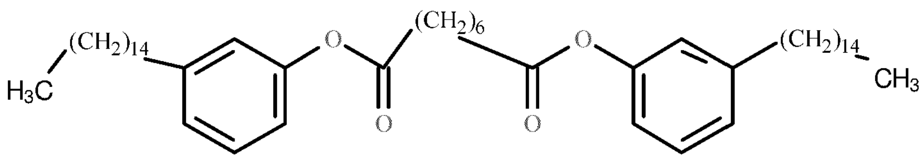

(14)

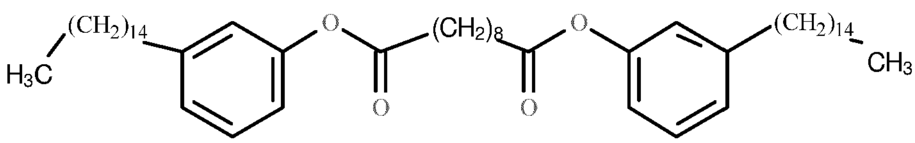

(15)

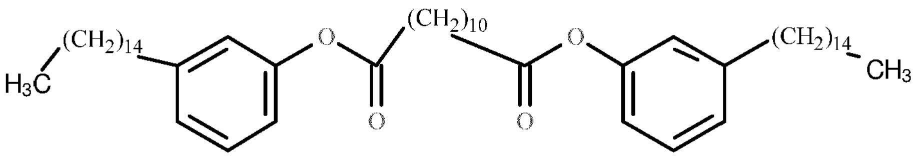

(16)

6. Composition de pigment thermochrome comprenant :

(A) au moins un composé organique colorant donneur d'électrons,
(B) au moins un composé accepteur d'électrons, et
(C) au moins un composé répondant à la formule (I) suivante :

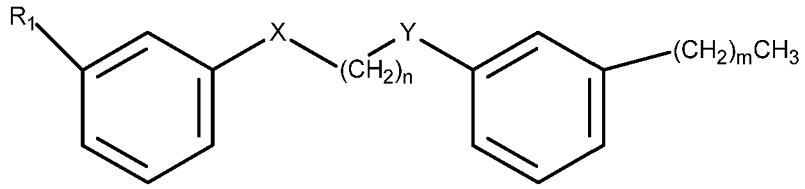

(I)

dans laquelle :

- X représente $CHR_2$, O, OCO ou CH=CH,
- Y représente O ou COO,
- $R_1$ représente H ou $(CH_2)_pCH_3$,
- $R_2$ représente un groupe phényle ou H,
- m = 12-18,
- n = 0-14,
- p = 12-18, et

à la condition que lorsque n = 0, X représente $CHR_2$ ou CH=CH.

7. Composition selon la revendication 6, dans laquelle le composé (A) est choisi parmi le 3-(4-diéthylamino-2-éthoxyphényl)-3-(1-éthyl-2-méthylindol-3-yl)-4-azaphthalide (Blue 63, n° CAS: 69898-40-4), le 2'-(dibenzylamino)-6'-(diéthylamino)fluorane (n° CAS: 34372-72-0), le N,N-diméthyl-4-[2-[2-(octyloxy)phényl]-6-phényl-4-pyridinyl]benzènamine (Yellow CK37, n° CAS : 144190-25-0), le 7-(4-diéthylamino-2-hexyloxyphényl)-7-(1-éthyl-2-méthyl-1H-indol-3-yl)-7H-furo[3,4-b]pyridin-5-one (Blue 203, N° CAS : 98660-18-5), le 2-(2,4-diméthylphénylamino)-3-méthyl-6-diéthylaminofluoran (Black 15, N° CAS: 36431-22-8), et le 3,3-bis-(1-butyl-2-méthyl-indol-3-yl)-3H-isobenzofuran-1-one (Red 40, n° CAS : 50292-91-6).

8. Composition selon la revendication 6 ou la revendication 7, dans laquelle le composé (B) est choisi parmi le 2,2-bis(4-hydroxy-3-méthylphényl)propane (Bisphénol C, n° CAS: 79-97-0), le 4-hexyl-1,3-dihydroxybenzène (4-hexylrésorcinol, n° CAS : 136-77-6), le 4,4'-cyclohexylidènebisphénol (BPZ, n° CAS: 843-55-0), le 4,4'-(hexafluoroisopropylidène)diphénol (Bisphénol AF, n° CAS: 1478-61-1), le 4,4'-(1-phényléthylidène)bisphénol (n° CAS: 1571-75-1), le 2,2'-dihydroxybiphényl (n° CAS : 1806-29-7), le 4,4'-(1,4-phénylènediisopropylidène)bisphénol (n° CAS: 2167-51-3), le 1,1-bis(4-hydroxy-3-méthylphényl)cyclohexane (n° CAS: 2362-14-3), le 9,9-bis(4-hydroxyphényl)fluorène (n° CAS : 3236-71-3), le 4,4'-(1,3-phénylènediisopropylidène)bisphénol (n° CAS: 13595-25-0), le 1,1,1-tris(4-hydroxyphényl)éthane (n° CAS : 27955-94-8), le 4,4'-(2-éthylhexylidène)diphénol (n° CAS: 74462-02-5), le a,a,a'-tris(4-hydroxyphényl)-1-éthyl-4-isopropylbenzène (n° CAS : 110726-28-8).

9. Microcapsule de pigment thermochrome comprenant une composition selon l'une des revendications 6 à 8.

10. Composition d'encre comprenant des microcapsules de pigment thermochrome selon la revendication 9.

11. Instrument d'écriture comprenant une composition d'encre selon la revendication 10.

12. Instrument d'écriture selon la revendication 11 choisi parmi les stylos à bille à encre effaçable par friction.

**Patentansprüche**

1. Verbindung, die der folgenden Formel (I) entspricht:

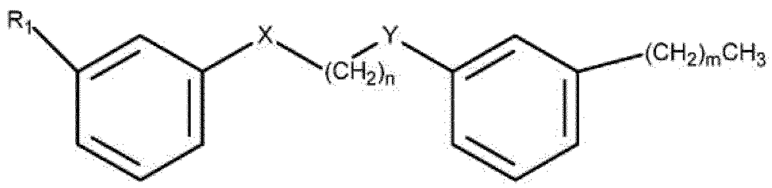

(I)

wobei:

- X für $CHR_2$, O oder OCO steht,
- Y für O oder COO steht,
- $R_1$ für H oder $(CH_2)_pCH_3$ steht,
- $R_2$ für eine Phenylgruppe oder H steht,
- m = 12-18,
- n = 0-14,
- p = 12-18 und

unter der Voraussetzung, dass, wenn n = 0, X für $CHR_2$ steht.

**2.** Verbindung nach Anspruch 1, die der folgenden Formel ($I_a$) entspricht:

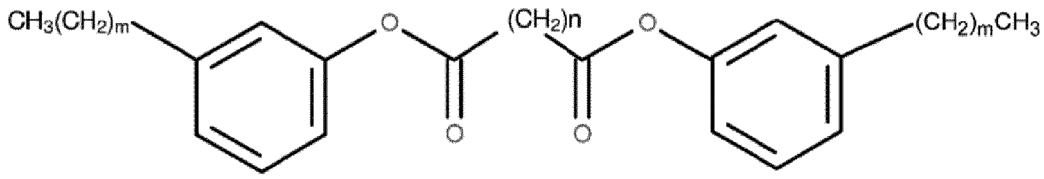

($I_a$)

wobei:

- m = 12-18 und
- n = 1-14.

**3.** Verbindung nach Anspruch 1, die der folgenden Formel ($I_b$) entspricht:

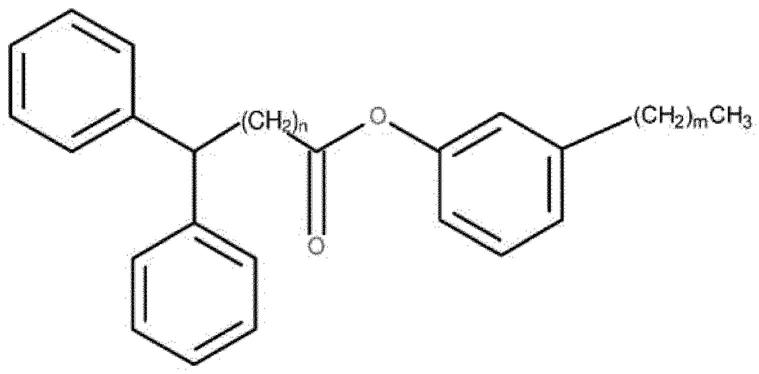

($I_b$)

wobei:

- m = 12-18 und

- n = 0-14.

**4.** Verbindung nach Anspruch 1, die der folgenden Formel ($I_c$) entspricht:

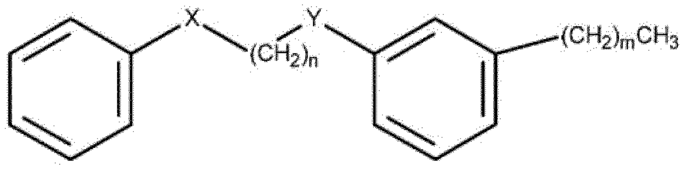

($I_c$)

wobei:

- X für $CH_2$ oder O steht,
- Y für O oder COO steht,
- m = 12-18 und
- n = 1-14.

**5.** Verbindung nach einem der Ansprüche 1 bis 4, ausgewählt aus einer der folgenden Verbindungen:

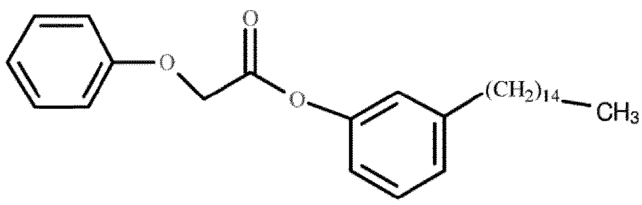

(1)

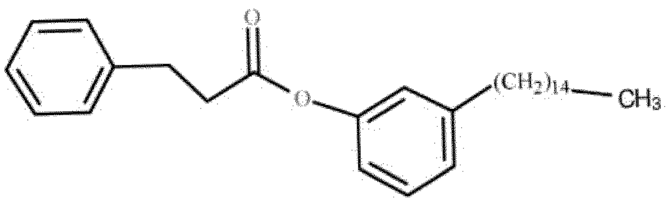

(3)

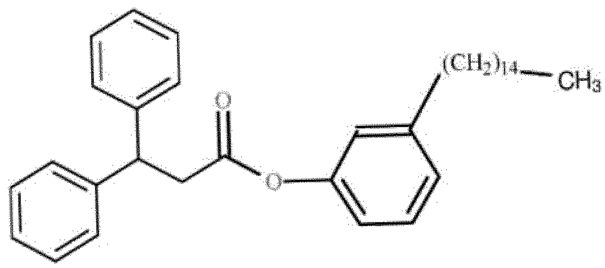

(4)

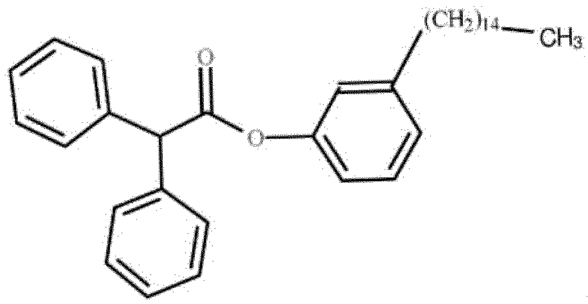

(5)

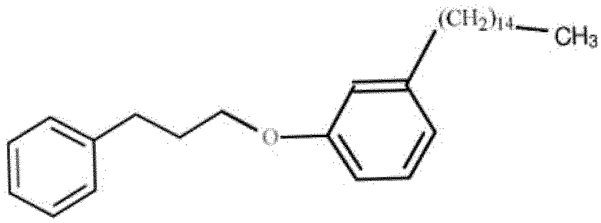

(6)

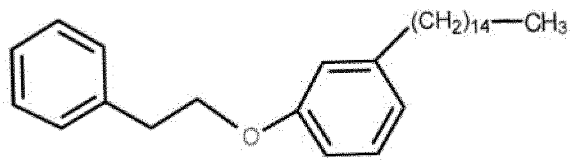

(7)

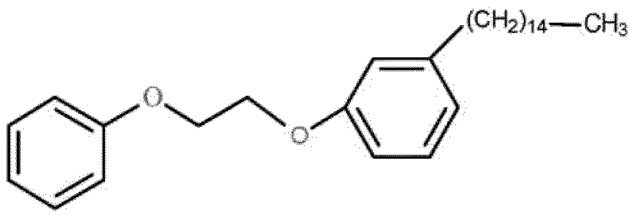

(8)

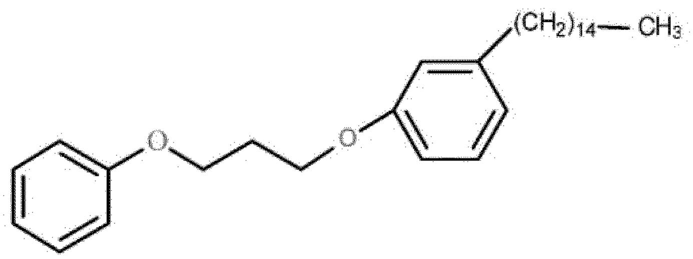

(9)

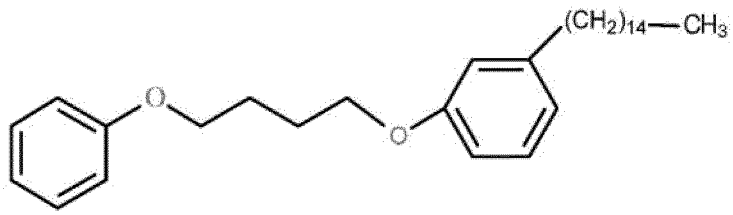

(10)

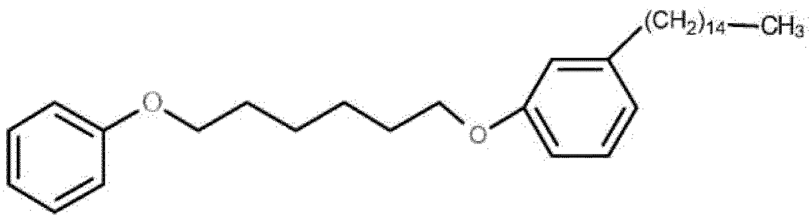

(11)

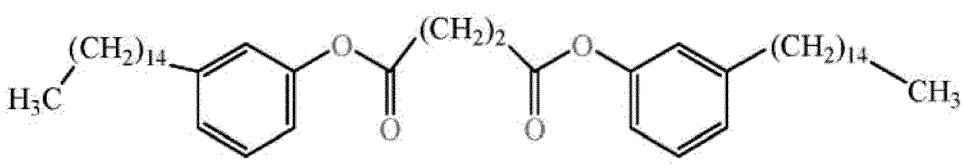

(12)

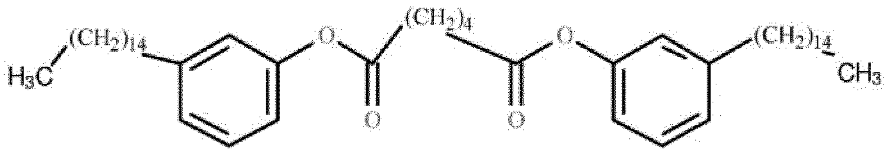

(13)

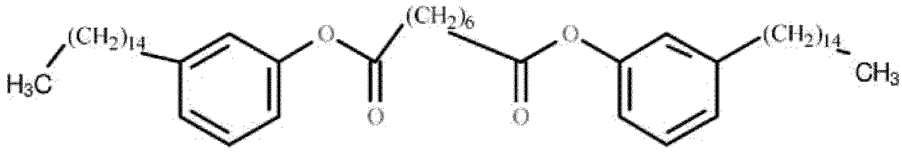

(14)

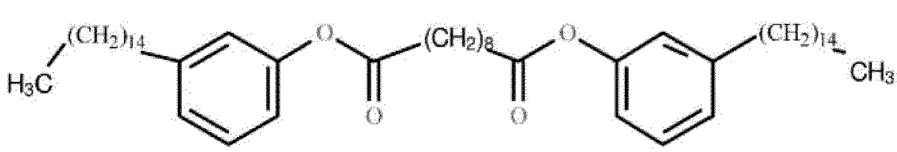

(15)

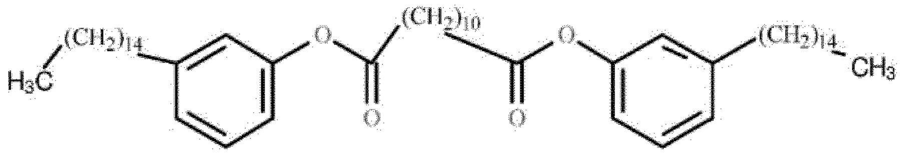

(16)

**6.** Thermochrome Pigmentzusammensetzung, umfassend:

(A) mindestens eine elektronenabgebende organische Farbstoffverbindung,
(B) mindestens eine elektronenaufnehmende Verbindung und
(C) mindestens eine Verbindung, die der folgenden Formel (I) entspricht:

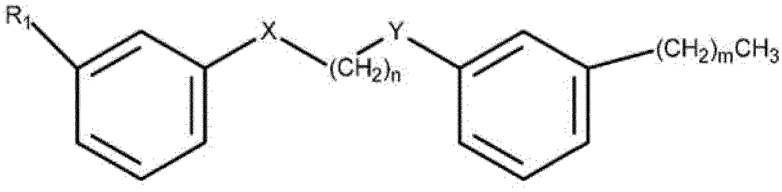

(I)

wobei:

- X für $CHR_2$, O, OCO oder CH=CH steht,
- Y für O oder COO steht,
- $R_1$ für H oder $(CH_2)_pCH_3$ steht,
- $R_2$ für eine Phenylgruppe oder H steht,
- m = 12-18,
- n = 0-14,
- p = 12-18 und

unter der Voraussetzung, dass, wenn n = 0, X für $CHR_2$ oder CH=CH steht.

**7.** Zusammensetzung nach Anspruch 6, wobei die Verbindung (A) aus 3-(4-Diethylamino-2-ethoxyphenyl)-3-(1-ethyl-2-methylindol-3-yl)-4-azaphthalid (Blue 63, CAS-Nr.: 69898-40-4), 2'-(Dibenzylamino)-6'-(diethylamino)fluoran (CAS-Nr.: 34372-72-0), N,N-Dimethyl-4-[2-[2-(octyloxy)phenyl]-6-phenyl-4-pyridinyl]benzolamin (Yellow CK37, CAS-Nr.: 144190-25-0), 7-(4-Diethylamino-2-hexyloxyphenyl)-7-(1-ethyl-2-methyl-1H-indol-3-yl)-7H-furo[3,4-b]pyridin-5-on (Blue 203, CAS-Nr.: 98660-18-5), 2-(2,4-Dimethylphenylamino)-3-methyl-6-diethylaminofluoran (Black 15, CAS-Nr.: 36431-22-8) und 3,3-Bis-(1-butyl-2-methyl-indol-3-yl)-3H-isobenzofuran-1-on (Red 40, CAS-Nr.: 50292-91-6) ausgewählt ist.

**8.** Zusammensetzung nach Anspruch 6 oder 7, wobei die Verbindung (B) aus 2,2-Bis(4-hydroxy-3-methylphenyl)propan (Bisphenol C, CAS-Nr.: 79-97-0), 4-Hexyl-1,3-dihydroxybenzol (4-Hexylresorcinol, CAS-Nr.: 136-77-6), 4,4'-Cyclohexylidenbisphenol (BPZ, CAS-Nr.: 843-55-0), 4,4'-(Hexafluorisopropyliden)diphenol (Bisphenol AF, CAS-Nr.: 1478-61-1), 4,4'-(1-Phenylethyliden)bisphenol (CAS-Nr.: 1571-75-1), 2,2'-Dihydroxybiphenyl (CAS-Nr.: 1806-29-7), 4,4'-(1,4-Phenylendiisopropyliden)bisphenol (CAS-Nr.: 2167-51-3), 1,1-Bis(4-hydroxy-3-methylphenyl)cyclohexan (CAS-Nr.: 2362-14-3), 9,9-Bis(4-hydroxyphenyl)fluoren (CAS-Nr.: 3236-71-3), 4,4'-(1,3-Phenylendiisopropyliden)bisphenol (CAS-Nr.: 13595-25-0), 1,1,1-Tris(4-hydroxyphenyl)ethan (CAS-Nr.: 27955-94-8), 4,4'-(2-Ethylhexyliden)diphenol (CAS-Nr.: 74462-02-5), $\alpha,\alpha,\alpha'$-Tris(4-hydroxyphenyl)-1-ethyl-4-isopropylbenzol (CAS-Nr.: 110726-28-8) ausgewählt ist.

**9.** Mikrokapsel des thermochromen Pigments, umfassend eine Zusammensetzung nach einem der Ansprüche 6 bis 8.

**10.** Tintenzusammensetzung, umfassend Mikrokapseln des thermochromen Pigments nach Anspruch 9.

**11.** Schreibgerät, umfassend eine Tintenzusammensetzung nach Anspruch 10.

**12.** Schreibgerät nach Anspruch 11, ausgewählt aus reibungslöschbaren Tintenkugelschreibern.

## Claims

**1.** Compound corresponding to the following formula (I):

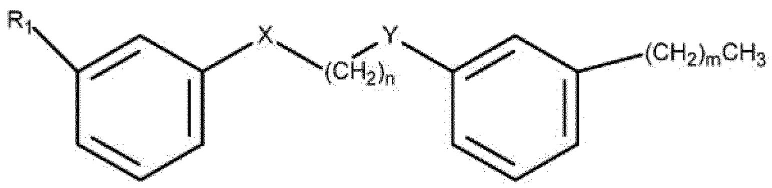

(I)

in which:

- X represents $CHR_2$, O or OCO,
- Y represents O or COO,
- $R_1$ represents H or $(CH_2)_pCH_3$,
- $R_2$ represents a phenyl group or H,
- m = 12-18,
- n = 0-14,
- p = 12-18, and

with the proviso that when n = 0, X represents $CHR_2$.

**2.** Compound according to claim 1 corresponding to the following formula ($I_a$):

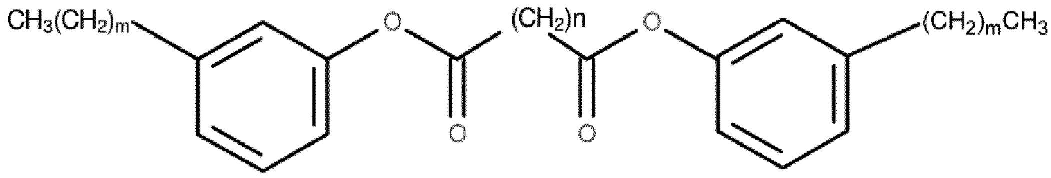

($I_a$)

in which:

- m = 12-18, and
- n = 1-14.

**3.** Compound according to claim 1 corresponding to the following formula ($I_b$):

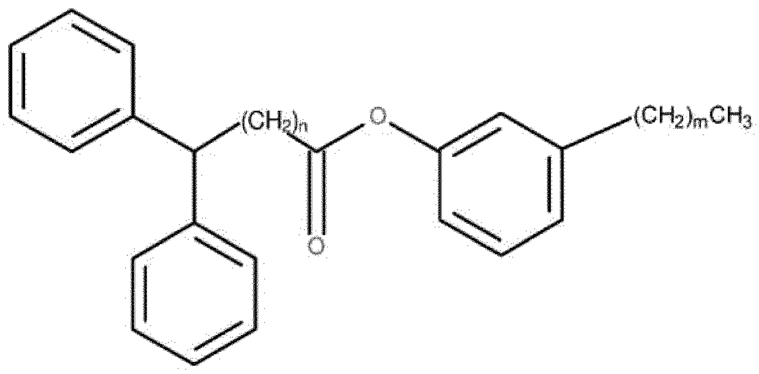

($I_b$)

in which:

- m = 12-18, and

- n = 0-14.

**4.** Compound according to claim 1 corresponding to the following formula ($I_c$):

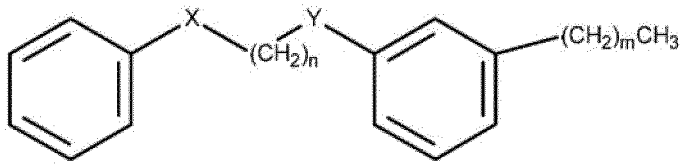

($I_c$)

in which:

- X represents $CH_2$ or O,
- Y represents O or COO,
- m = 12-18, and
- n = 1-14.

**5.** Compound according to any of claims 1 to 4 selected from one of the following compounds:

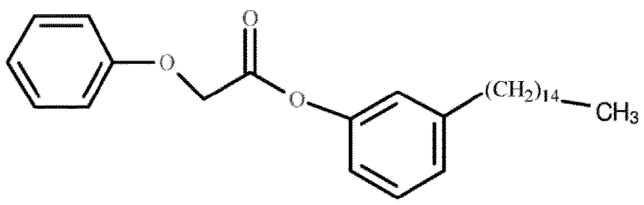

(1)

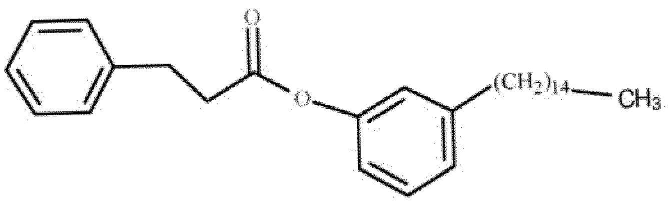

(3)

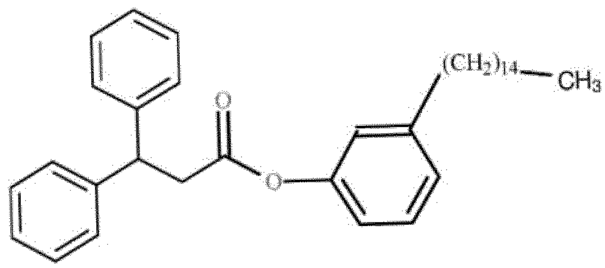

(4)

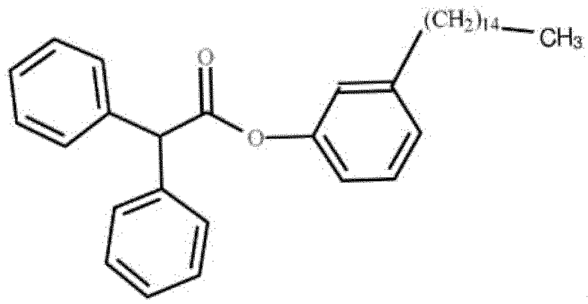

(5)

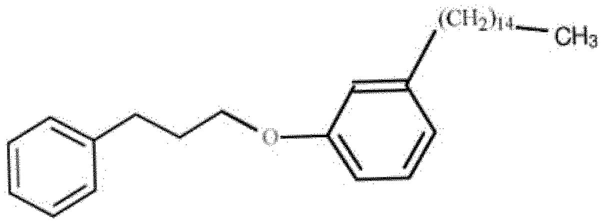

(6)

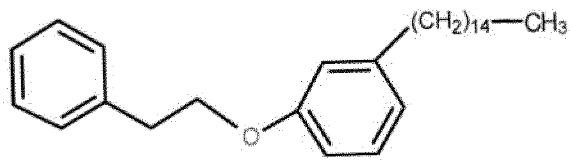

(7)

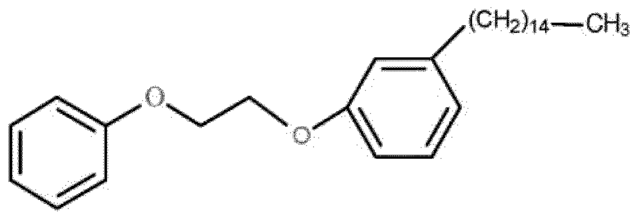

(8)

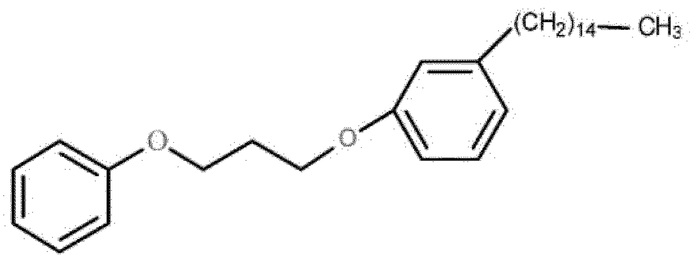

(9)

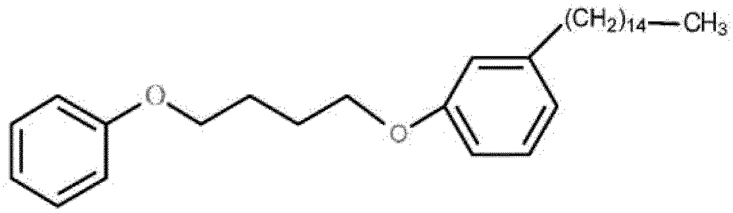

(10)

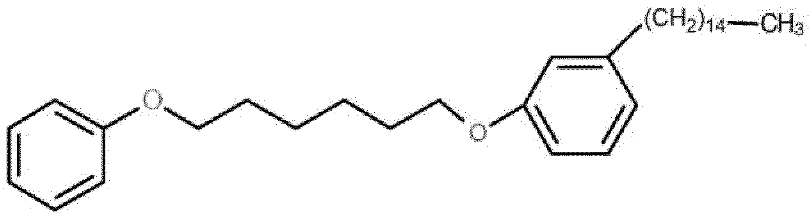

(11)

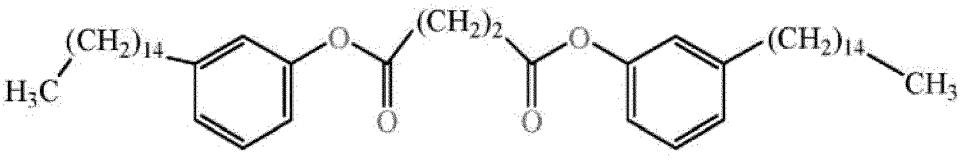

(12)

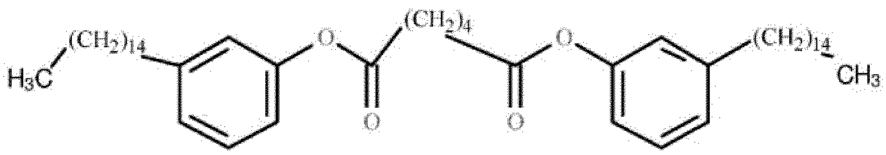

(13)

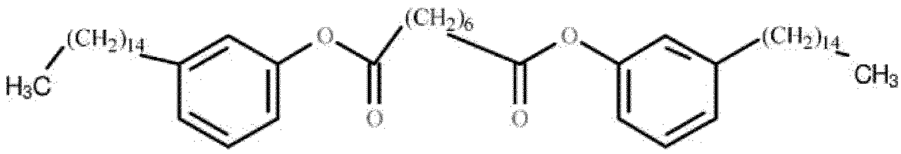

(14)

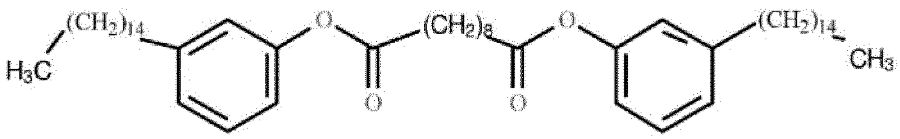

(15)

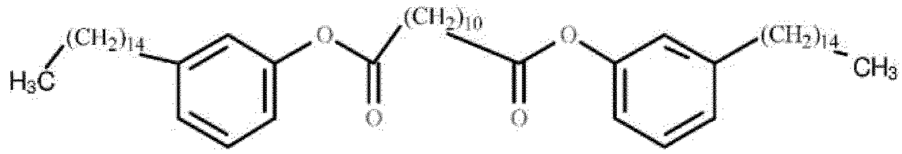

(16)

**6.** Thermochromic pigment composition comprising:

(A) at least one electron-donating organic dye compound,
(B) at least one electron-accepting compound, and
(C) at least one compound corresponding to the following formula (I):

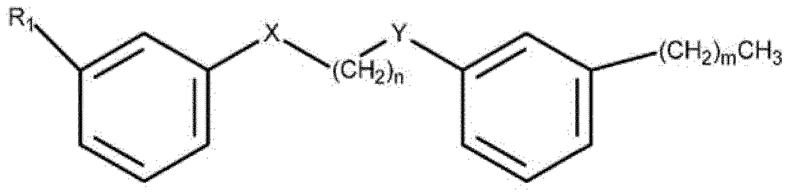

(I)

in which:

- X represents $CHR_2$, O, OCO or CH=CH,
- Y represents O or COO,
- $R_1$ represents H or $(CH_2)_pCH_3$,
- $R_2$ represents a phenyl group or H,
- m = 12-18,
- n = 0-14,
- p = 12-18, and

with the proviso that when n = 0, X represents $CHR_2$ or CH=CH.

**7.** Composition according to claim 6, wherein the compound (A) is selected from 3-(4-diethylamino-2-ethoxyphenyl)-3-(1-ethyl-2-methylindol-3-yl)-4-azaphthalide (Blue 63, CAS no.: 69898-40-4), 2'-(dibenzylamino)-6'-(diethylamino)fluoran (CAS no.: 34372-72-0), N,N-dimethyl-4-[2-[2-(octyloxy)phenyl]-6-phenyl-4-pyridinyl]benzenamine (Yellow CK37, CAS no.: 144190-25-0), 7-(4-diethylamino-2-hexyloxyphenyl)-7-(1-ethyl-2-methyl-1H-indol-3-yl)-7H-furo[3,4-b]pyridin-5-one (Blue 203, CAS no.: 98660-18-5), 2-(2,4-dimethylphenylamino)-3-methyl-6-diethylaminofluoran (Black 15, CAS no: 36431-22-8), and 3,3-bis-(1-butyl-2-methyl-indol-3-yl)-3H-isobenzofuran-1-one (Red 40, CAS no.: 50292-91-6).

**8.** Composition according to either claim 6 or claim 7, wherein the compound (B) is selected from 2,2-bis(4-hydroxy-3-methylphenyl)propane (Bisphenol C, CAS no.: 79-97-0), 4-hexyl-1,3-dihydroxybenzene (4-hexylresorcinol, CAS no.: 136-77-6), 4,4'-cyclohexylidenebisphenol (BPZ, CAS no.: 843-55-0), 4,4'-(hexafluoroisopropylidene)diphenol (Bisphenol AF, CAS no.: 1478-61-1), 4,4'-(1-phenylethylidene)bisphenol (CAS no.: 1571-75-1), 2,2'-dihydroxybiphenyl (CAS no.: 1806-29-7), 4,4'-(1,4-phenylenediisopropylidene)bisphenol (CAS no.: 2167-51-3), 1,1-bis(4-hydroxy-3-methylphenyl)cyclohexane (CAS no.: 2362-14-3), 9,9-bis(4-hydroxyphenyl)fluorene (CAS no.: 3236-71-3), 4,4'-(1,3-phenylenediisopropylidene)bisphenol (CAS no.: 13595-25-0), 1,1,1-tris(4-hydroxyphenyl)ethane (CAS no.: 27955-94-8), 4,4'-(2-ethylhexylidene)diphenol (CAS no.: 74462-02-5), $\alpha,\alpha,\alpha'$-tris(4-hydroxyphenyl)-1-ethyl-4-isopropylbenzene (CAS no.: 110726-28-8).

**9.** Thermochromic pigment microcapsule, comprising a composition according to any of claims 6 to 8.

**10.** Ink composition, comprising thermochromic pigment microcapsules according to claim 9.

**11.** Writing instrument comprising an ink composition according to claim 10.

**12.** Writing instrument according to claim 11, selected from friction-erasable ink ballpoint pens.

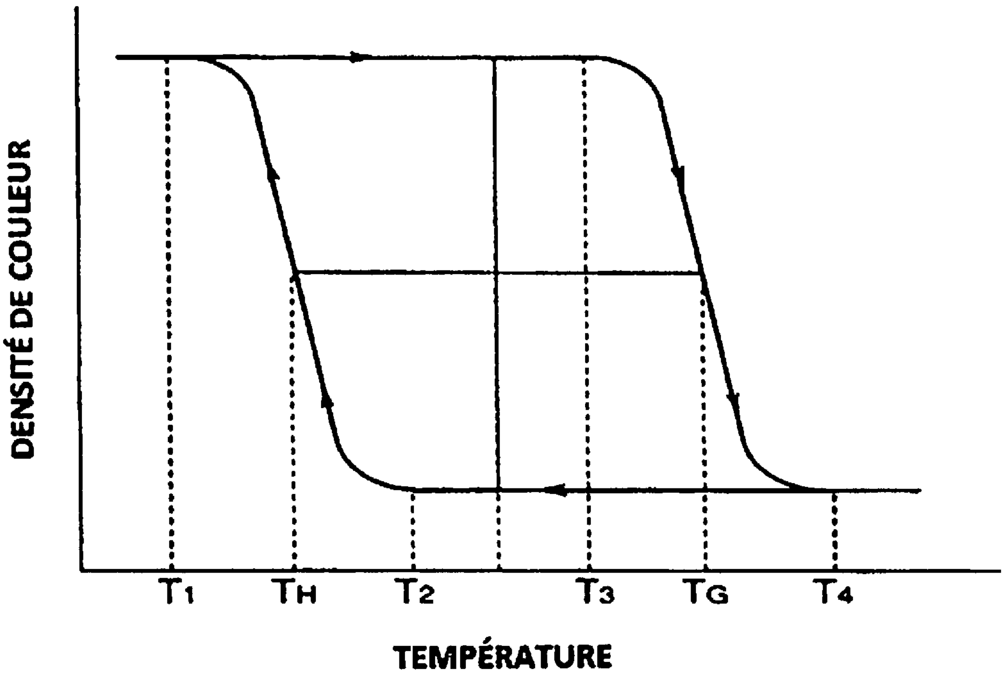


FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2016198784 A1

### Littérature non-brevet citée dans la description

- *CHEMICAL ABSTRACTS,* 69898-40-4
- *CHEMICAL ABSTRACTS,* 1552-42-7
- *CHEMICAL ABSTRACTS,* 21121-62-0
- *CHEMICAL ABSTRACTS,* 21934-68-9
- *CHEMICAL ABSTRACTS,* 26567-23-7
- *CHEMICAL ABSTRACTS,* 26628-47-7
- *CHEMICAL ABSTRACTS,* 29199-09-5
- *CHEMICAL ABSTRACTS,* 29512-49-0
- *CHEMICAL ABSTRACTS,* 34372-72-0
- *CHEMICAL ABSTRACTS,* 36431-22-8
- *CHEMICAL ABSTRACTS,* 36499-49-7
- *CHEMICAL ABSTRACTS,* 36886-76-7
- *CHEMICAL ABSTRACTS,* 50292-91-6
- *CHEMICAL ABSTRACTS,* 50292-95-0
- *CHEMICAL ABSTRACTS,* 59129-79-2
- *CHEMICAL ABSTRACTS,* 70516-41-5
- *CHEMICAL ABSTRACTS,* 75805-17-3
- *CHEMICAL ABSTRACTS,* 82137-81-3
- *CHEMICAL ABSTRACTS,* 89331-94-2
- *CHEMICAL ABSTRACTS,* 92453-31-1
- *CHEMICAL ABSTRACTS,* 98660-18-5
- *CHEMICAL ABSTRACTS,* 132467-74-4
- *CHEMICAL ABSTRACTS,* 144190-25-0
- *CHEMICAL ABSTRACTS,* 148716-90-9
- *CHEMICAL ABSTRACTS,* 79-97-0
- *CHEMICAL ABSTRACTS,* 136-77-6
- *CHEMICAL ABSTRACTS,* 843-55-0
- *CHEMICAL ABSTRACTS,* 1478-61-1
- *CHEMICAL ABSTRACTS,* 1571-75-1
- *CHEMICAL ABSTRACTS,* 1806-29-7
- *CHEMICAL ABSTRACTS,* 2081-08-5
- *CHEMICAL ABSTRACTS,* 2167-51-3
- *CHEMICAL ABSTRACTS,* 2362-14-3
- *CHEMICAL ABSTRACTS,* 3236-71-3
- *CHEMICAL ABSTRACTS,* 13595-25-0
- *CHEMICAL ABSTRACTS,* 27955-94-8
- *CHEMICAL ABSTRACTS,* 74462-02-5
- *CHEMICAL ABSTRACTS,* 110726-28-8
- *CHEMICAL ABSTRACTS,* 140-66-9
- *CHEMICAL ABSTRACTS,* 831-82-3
- *CHEMICAL ABSTRACTS,* 1096-84-0
- *CHEMICAL ABSTRACTS,* 14763-60-1
- *CHEMICAL ABSTRACTS,* 95235-30-6
- *CHEMICAL ABSTRACTS,* 92-88-6
- *CHEMICAL ABSTRACTS,* 92-69-3
- *CHEMICAL ABSTRACTS,* 99-89-8
- *CHEMICAL ABSTRACTS,* 131-56-6
- *CHEMICAL ABSTRACTS,* 150-76-5
- *CHEMICAL ABSTRACTS,* 501-24-6
- *CHEMICAL ABSTRACTS,* 599-64-4
- *CHEMICAL ABSTRACTS,* 3380-34-5
- *CHEMICAL ABSTRACTS,* 232938-43-1
- *CHEMICAL ABSTRACTS,* 79-94-7
- *CHEMICAL ABSTRACTS,* 80-05-7
- *CHEMICAL ABSTRACTS,* 80-09-1
- *CHEMICAL ABSTRACTS,* 637-59-2
- *CHEMICAL ABSTRACTS,* 584-08-7
- *CHEMICAL ABSTRACTS,* 1310-73-2
- *CHEMICAL ABSTRACTS,* 103-63-9
- *CHEMICAL ABSTRACTS,* 589-10-6
- *CHEMICAL ABSTRACTS,* 588-63-6
- *CHEMICAL ABSTRACTS,* 1200-03-9
- *CHEMICAL ABSTRACTS,* 57795-97-2
- *CHEMICAL ABSTRACTS,* 121-44-8
- *CHEMICAL ABSTRACTS,* 701-99-5
- *CHEMICAL ABSTRACTS,* 109-99-9
- *CHEMICAL ABSTRACTS,* 102-92-1
- *CHEMICAL ABSTRACTS,* 501-52-0

- *CHEMICAL ABSTRACTS,* 6192-52-5
- *CHEMICAL ABSTRACTS,* 606-83-7
- *CHEMICAL ABSTRACTS,* 1871-76-7
- *CHEMICAL ABSTRACTS,* 543-20-4
- *CHEMICAL ABSTRACTS,* 111-50-2
- *CHEMICAL ABSTRACTS,* 10027-07-3
- *CHEMICAL ABSTRACTS,* 111-19-3
- *CHEMICAL ABSTRACTS,* 4834-98-4